(19) Europäisches Patentamt
European Patent Office
Office européen des brevets

(11) EP 2 374 464 A2

(12) EUROPEAN PATENT APPLICATION

(43) Date of publication:
**12.10.2011 Bulletin 2011/41**

(21) Application number: **11157671.6**

(22) Date of filing: **30.09.2005**

(51) Int Cl.:
***A61K 38/00*** *(2006.01)* ***A61K 31/4965*** *(2006.01)*
***A61K 31/42*** *(2006.01)* ***A61K 31/425*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **01.10.2004 US 615412 P**

(62) Document number(s) of the earlier application(s) in accordance with Art. 76 EPC:
**05808364.3 / 1 804 821**

(71) Applicant: **VERTEX PHARMACEUTICALS INCORPORATED**
**Cambridge, MA 02139-4242 (US)**

(72) Inventor: **Lin, Chao**
**Wlinchester,MA 01890 (US)**

(74) Representative: **Cabinet Plasseraud**
**52, rue de la Victoire**
**75440 Paris Cedex 09 (FR)**

Remarks:
This application was filed on 10-03-2011 as a divisional application to the application mentioned under INID code 62.

# (54) HCV N3S-NS4A protease inhibition

(57) The present invention relates to inhibiting the activity of non-genotype 1 hepatitis C virus (HCV) NS3-NS4A protease activity. More particularly, the invention relates to inhibiting the activity of the protease from HCV genotype-2 or HCV genotype 3. The methods of the invention imply inhibitors such as VX-950 that act by interfering with the life cycle of the HCV and are also useful as antiviral agents. The invention further relates to compositions comprising such compounds either for *ex vivo* use or for administration to a patient suffering from genotype-2 or genotype-3 HCV infection. The invention also relates to methods of treating an HCV infection in a patient by administering a composition comprising a compound of this invention.



Printed by Jouve, 75001 PARIS (FR)

SEQUENCE LISTING

<110> Lin, et al

<120> HCV NS3-NS4A Protease Inhibition

<130> 30852/40507

<140> PCT/US2005/035191
<141> 2005-09-30

<150> 60/615,412
<151> 2004-10-01

<160> 33

<170> PatentIn version 3.3

<210> 1
<211> 543
<212> DNA
<213> Hepatitis C virus

<400> 1
gcccccatca ctgcttatgc ccagcagaca cgcggccttt tgggcgccat agtggtgagc 60

atgacggggc gcgacaagac agaacaggcc ggggagattc aggtcctgtc cacggtcact 120

cagtccttcc tcggaacaac catctcgggg gtcttatgga ctgtctacca tggagctggc 180

aacaagactc tagccggctc acggggtccg gtcacacaga tgtactccag tgctgagggg 240

gacttagtgg ggtggcccag cccccccggg accaaatctt tggagccgtg cacgtgtgga 300

gcggtcgacc tatacctggt cacgcgaaac gctgatgtca tcccggctcg aagacgcggg 360

gacaagcgag gagcgctact ctccccgaga cctctttcca ccttgaaggg gtcctcgggg 420

ggcccggtgc tctgccccag aggccacgct gtcgggatct tccgggcagc cgtgtgctcc 480

cggggcgtgg ccaagtccat agattttatc cccgttgaga cacttgacat cgtcactcgg 540

tcc 543

<210> 2
<211> 181
<212> PRT
<213> Hepatitis C virus

<400> 2

Ala Pro Ile Thr Ala Tyr Ala Gln Gln Thr Arg Gly Leu Leu Gly Ala
1 5 10 15

Ile Val Val Ser Met Thr Gly Arg Asp Lys Thr Glu Gln Ala Gly Glu
20 25 30

Ile Gln Val Leu Ser Thr Val Thr Gln Ser Phe Leu Gly Thr Thr Ile
35 40 45

Ser Gly Val Leu Trp Thr Val Tyr His Gly Ala Gly Asn Lys Thr Leu
50 55 60

```
Ala Gly Ser Arg Gly Pro Val Thr Gln Met Tyr Ser Ser Ala Glu Gly
65                  70                  75                  80

Asp Leu Val Gly Trp Pro Ser Pro Pro Gly Thr Lys Ser Leu Glu Pro
                85                  90                  95

Cys Thr Cys Gly Ala Val Asp Leu Tyr Leu Val Thr Arg Asn Ala Asp
            100                 105                 110

Val Ile Pro Ala Arg Arg Arg Gly Asp Lys Arg Gly Ala Leu Leu Ser
        115                 120                 125

Pro Arg Pro Leu Ser Thr Leu Lys Gly Ser Ser Gly Gly Pro Val Leu
    130                 135                 140

Cys Pro Arg Gly His Ala Val Gly Ile Phe Arg Ala Ala Val Cys Ser
145                 150                 155                 160

Arg Gly Val Ala Lys Ser Ile Asp Phe Ile Pro Val Glu Thr Leu Asp
                165                 170                 175

Ile Val Thr Arg Ser
            180

<210>  3
<211>  181
<212>  PRT
<213>  Hepatitis C virus

<400>  3

Ala Pro Ile Thr Ala Tyr Ala Gln Gln Thr Arg Gly Leu Leu Gly Ala
1               5                   10                  15

Ile Val Val Ser Met Thr Gly Arg Asp Lys Thr Glu Gln Ala Gly Glu
            20                  25                  30

Ile Gln Val Leu Ser Thr Val Thr Gln Ser Phe Leu Gly Ser Thr Ile
        35                  40                  45

Ser Gly Val Leu Trp Thr Val Tyr His Gly Ala Gly Asn Lys Thr Leu
    50                  55                  60

Ala Gly Ser Arg Gly Pro Val Thr Gln Met Tyr Ser Ser Ala Glu Gly
65                  70                  75                  80

Asp Leu Val Gly Trp Pro Ser Pro Pro Gly Thr Lys Ser Leu Glu Pro
                85                  90                  95

Cys Thr Cys Gly Ala Val Asp Leu Tyr Leu Val Thr Arg Asn Ala Asp
            100                 105                 110
```

```
Val Ile Pro Ala Arg Arg Arg Gly Asp Lys Arg Gly Ala Leu Leu Ser
        115                 120                 125

Pro Arg Pro Leu Ser Thr Leu Lys Gly Ser Ser Gly Gly Pro Val Leu
    130                 135                 140

Cys Pro Arg Gly His Ala Val Gly Ile Phe Arg Ala Ala Val Cys Ser
145                 150                 155                 160

Arg Gly Val Ala Lys Ser Ile Asp Phe Ile Pro Val Glu Ser Leu Asp
                165                 170                 175

Ile Val Thr Arg Ser
            180

<210>  4
<211>  181
<212>  PRT
<213>  Hepatitis C virus

<400>  4

Ala Pro Ile Thr Ala Tyr Ala Gln Gln Thr Arg Gly Leu Leu Gly Thr
  1               5                  10                  15

Ile Val Val Ser Met Thr Gly Arg Asp Lys Thr Glu Gln Ala Gly Glu
             20                  25                  30

Ile Gln Val Leu Ser Thr Val Thr Gln Ser Phe Leu Gly Thr Thr Ile
         35                  40                  45

Ser Gly Val Leu Trp Thr Val Tyr His Gly Ala Gly Asn Lys Thr Leu
     50                  55                  60

Ala Gly Ser Arg Gly Pro Val Thr Gln Met Tyr Ser Ser Ala Glu Gly
 65                  70                  75                  80

Asp Leu Val Gly Trp Pro Ser Pro Pro Gly Thr Lys Ser Leu Glu Pro
                 85                  90                  95

Cys Thr Cys Gly Ala Val Asp Leu Tyr Leu Val Thr Arg Asn Ala Asp
            100                 105                 110

Val Ile Pro Ala Arg Arg Arg Gly Asp Lys Arg Gly Ala Leu Leu Ser
        115                 120                 125

Pro Arg Pro Leu Ser Thr Leu Lys Gly Ser Ser Gly Gly Pro Val Leu
    130                 135                 140

Cys Pro Arg Gly His Ala Val Gly Val Phe Arg Ala Ala Val Cys Ser
145                 150                 155                 160

Arg Gly Val Ala Lys Ser Ile Asp Phe Ile Pro Val Glu Thr Leu Asp
```

165 170 175

Ile Val Thr Arg Ser
180

<210> 5
<211> 181
<212> PRT
<213> Hepatitis C virus

<400> 5

Ala Pro Ile Thr Ala Tyr Ala Gln Gln Thr Arg Gly Leu Leu Gly Thr
1 5 10 15

Ile Val Val Ser Met Thr Gly Arg Asp Lys Thr Glu Gln Ala Gly Glu
20 25 30

Ile Gln Val Leu Ser Thr Val Thr Gln Ser Phe Leu Gly Thr Thr Ile
35 40 45

Ser Gly Val Leu Trp Thr Val Tyr His Gly Ala Gly Asn Lys Thr Leu
50 55 60

Ala Gly Ser Arg Gly Pro Val Thr Gln Met Tyr Ser Ser Ala Glu Gly
65 70 75 80

Asp Leu Val Gly Trp Pro Ser Pro Pro Gly Thr Lys Ser Leu Glu Pro
85 90 95

Cys Thr Cys Gly Ala Val Asp Leu Tyr Leu Val Thr Arg Asn Ala Asp
100 105 110

Val Ile Pro Ala Arg Arg Arg Gly Asp Lys Arg Gly Ala Leu Leu Ser
115 120 125

Pro Arg Pro Leu Ser Thr Leu Lys Gly Ser Ser Gly Gly Pro Val Leu
130 135 140

Cys Pro Arg Gly His Ala Val Gly Val Phe Arg Ala Ala Val Cys Ser
145 150 155 160

Arg Gly Val Ala Lys Ser Ile Asp Phe Ile Pro Val Glu Thr Leu Asp
165 170 175

Ile Val Thr Arg Ser
180

<210> 6
<211> 181
<212> PRT
<213> Hepatitis C virus

<400> 6

Ala Pro Ile Thr Ala Tyr Ala Gln Gln Thr Arg Gly Leu Leu Gly Thr
1               5                   10                  15

Ile Val Val Ser Met Thr Gly Arg Asp Lys Thr Glu Gln Ala Gly Glu
            20                  25                  30

Ile Gln Val Leu Ser Thr Val Thr Gln Ser Phe Leu Gly Thr Thr Ile
        35                  40                  45

Ser Gly Ile Leu Trp Thr Val Phe His Gly Ala Gly Asn Lys Thr Leu
    50                  55                  60

Ala Gly Ser Arg Gly Pro Val Thr Gln Met Tyr Ser Ser Ala Glu Gly
65                  70                  75                  80

Asp Leu Val Gly Trp Pro Ser Pro Pro Gly Thr Arg Ser Leu Asp Pro
                85                  90                  95

Cys Thr Cys Gly Ala Val Asp Leu Tyr Leu Val Thr Arg Asn Ala Asp
            100                 105                 110

Val Ile Pro Ala Arg Arg Gln Gly Asp Arg Arg Gly Ala Leu Leu Ser
        115                 120                 125

Pro Arg Pro Leu Ser Ser Leu Lys Gly Ser Ser Gly Gly Pro Val Leu
    130                 135                 140

Cys Pro Arg Gly His Ala Val Gly Ile Phe Arg Ala Ala Ile Cys Thr
145                 150                 155                 160

Arg Gly Ala Ala Lys Ser Ile Asp Phe Ile Pro Ile Glu Thr Leu Asp
                165                 170                 175

Val Ile Ile Arg Ser
            180

<210> 7
<211> 181
<212> PRT
<213> Hepatitis C virus

<400> 7

Ala Pro Ile Thr Ala Tyr Ala Gln Gln Thr Arg Gly Leu Leu Ser Ala
1               5                   10                  15

Ile Val Val Ser Met Thr Gly Arg Asp Lys Thr Asp Gln Ala Gly Glu
            20                  25                  30

Ile Gln Val Leu Ser Thr Val Thr Gln Ser Phe Leu Gly Thr Thr Ile
        35                  40                  45

Ser Gly Val Leu Trp Thr Val Phe His Gly Ala Gly Asn Lys Thr Leu
    50                  55                  60

Ala Gly Ser Arg Gly Pro Val Thr Gln Met Tyr Ser Ser Ala Glu Gly
65 70 75 80

Asp Leu Val Gly Trp Pro Ser Pro Pro Gly Thr Arg Ser Leu Glu Pro
85 90 95

Cys Thr Cys Gly Ala Val Asp Leu Tyr Leu Val Thr Arg Asn Ala Asp
100 105 110

Val Ile Pro Ala Arg Arg Arg Gly Asp Arg Arg Gly Ala Leu Leu Ser
115 120 125

Pro Arg Pro Leu Ser Ser Leu Lys Gly Ser Ser Gly Gly Pro Val Leu
130 135 140

Cys Pro Arg Gly His Ala Val Gly Ile Phe Arg Ala Ala Val Cys Ser
145 150 155 160

Arg Gly Val Ala Lys Ser Ile Asp Phe Ile Pro Val Glu Thr Leu Asp
165 170 175

Val Val Thr Arg Ser
180

<210> 8
<211> 181
<212> PRT
<213> Hepatitis C virus

<400> 8

Ala Pro Ile Thr Ala Tyr Ala Gln Gln Thr Arg Gly Leu Leu Gly Ala
1 5 10 15

Ile Val Val Ser Met Thr Gly Arg Asp Lys Thr Glu Gln Ala Gly Glu
20 25 30

Ile Gln Val Leu Ser Thr Val Thr Gln Ser Phe Leu Gly Thr Thr Ile
35 40 45

Ser Gly Val Leu Trp Thr Val Tyr His Gly Ala Gly Asn Lys Thr Leu
50 55 60

Ala Gly Ser Arg Gly Pro Val Thr Gln Met Tyr Ser Ser Ala Glu Gly
65 70 75 80

Asp Leu Val Gly Trp Pro Ser Pro Pro Gly Thr Lys Ser Leu Glu Pro
85 90 95

Cys Thr Cys Gly Ala Val Asp Leu Tyr Leu Val Thr Arg Asn Ala Asp
100 105 110

```
Val Ile Pro Ala Arg Arg Arg Gly Asp Lys Arg Gly Ala Leu Leu Ser
        115                 120                 125

Pro Arg Pro Leu Ser Thr Leu Lys Gly Ser Ser Gly Gly Pro Val Leu
    130                 135                 140

Cys Pro Arg Gly His Ala Val Gly Ile Phe Arg Ala Ala Val Cys Ser
145                 150                 155                 160

Arg Gly Val Ala Lys Ser Ile Asp Phe Ile Pro Val Glu Thr Leu Asp
                165                 170                 175

Ile Val Thr Arg Ser
            180


<210>  9
<211>  181
<212>  PRT
<213>  Hepatitis C virus


<220>
<221>  misc_feature
<222>  (132)..(132)
<223>  Xaa can be any naturally occurring amino acid

<400>  9

Ala Pro Ile Thr Ala Tyr Ala Gln Gln Thr Arg Gly Leu Leu Gly Ala
1               5                   10                  15

Ile Val Val Ser Met Thr Gly Arg Asp Lys Thr Glu His Ala Gly Glu
            20                  25                  30

Ile Gln Val Leu Ser Thr Val Thr Gln Ser Phe Leu Gly Thr Thr Ile
        35                  40                  45

Ser Gly Ile Leu Trp Thr Val Tyr His Gly Ala Gly Asn Lys Thr Leu
    50                  55                  60

Ala Gly Ser Arg Gly Pro Val Thr Gln Met Tyr Ser Ser Ala Glu Gly
65                  70                  75                  80

Asp Leu Val Gly Trp Pro Ser Pro Pro Gly Thr Lys Ser Leu Glu Pro
                85                  90                  95

Cys Thr Cys Gly Ala Val Asp Leu Tyr Leu Val Thr Arg Asn Ala Asp
            100                 105                 110

Val Ile Pro Ala Arg Arg Arg Gly Asp Lys Arg Gly Ala Leu Leu Ser
        115                 120                 125

Pro Arg Pro Xaa Ser Thr Leu Lys Gly Ser Ser Gly Gly Pro Val Leu
    130                 135                 140
```

Cys Pro Arg Gly His Ala Val Gly Ile Phe Arg Ala Ala Val Cys Ser
145 150 155 160

Arg Gly Val Ala Lys Ser Ile Asp Phe Ile Pro Val Glu Ala Leu Asp
165 170 175

Ile Val Thr Arg Ser
180

<210> 10
<211> 181
<212> PRT
<213> Hepatitis C virus

<400> 10

Ala Pro Ile Thr Ala Tyr Ala Gln Gln Thr Arg Gly Leu Leu Gly Ala
1 5 10 15

Ile Val Val Ser Met Thr Gly Arg Asp Lys Thr Glu Gln Ala Gly Glu
20 25 30

Ile Gln Ile Leu Ser Thr Val Thr Gln Ser Phe Leu Gly Thr Thr Ile
35 40 45

Ala Gly Val Leu Trp Thr Val Tyr His Gly Ala Gly Asn Lys Thr Leu
50 55 60

Ala Gly Ser Arg Gly Pro Val Thr Gln Met Tyr Ser Ser Ala Glu Gly
65 70 75 80

Asp Leu Val Gly Trp Pro Ser Pro Pro Gly Thr Lys Ser Leu Glu Pro
85 90 95

Cys Thr Cys Gly Ala Val Asp Leu Tyr Leu Val Thr Arg Asn Ala Asp
100 105 110

Val Ile Pro Ala Arg Arg Arg Gly Asp Lys Arg Gly Ala Leu Leu Ser
115 120 125

Pro Arg Pro Leu Ser Thr Leu Lys Gly Ser Ser Gly Gly Pro Val Leu
130 135 140

Cys Pro Arg Gly His Ala Val Gly Ile Phe Arg Ala Ala Val Cys Ser
145 150 155 160

Arg Gly Val Ala Lys Ser Ile Asp Phe Ile Pro Val Glu Thr Leu Asp
165 170 175

Ile Val Thr Arg Ser
180

<210> 11
<211> 181

<212> PRT
<213> Hepatitis C virus

<400> 11

```
Ala Pro Ile Thr Ala Tyr Ala Gln Gln Thr Arg Gly Leu Leu Gly Ala
1               5                   10                  15

Ile Val Val Ser Met Thr Gly Arg Asp Lys Thr Glu Gln Ala Gly Glu
            20                  25                  30

Ile Gln Val Leu Ser Thr Val Thr Gln Ser Phe Leu Gly Thr Thr Ile
        35                  40                  45

Ser Gly Val Leu Trp Thr Val Tyr His Gly Ala Gly Asn Lys Thr Leu
    50                  55                  60

Ala Gly Ser Arg Gly Pro Val Thr Gln Met Tyr Ser Ser Ala Glu Gly
65                  70                  75                  80

Asp Leu Val Gly Trp Pro Ser Pro Pro Gly Thr Lys Ser Leu Glu Pro
                85                  90                  95

Cys Thr Cys Gly Ala Val Asp Leu Tyr Leu Val Thr Arg Asn Ala Asp
            100                 105                 110

Val Ile Pro Ala Arg Arg Arg Gly Asp Lys Arg Gly Ala Leu Leu Ser
        115                 120                 125

Pro Arg Pro Leu Ser Thr Leu Lys Gly Ser Ser Gly Gly Pro Val Leu
    130                 135                 140

Cys Pro Arg Gly His Ala Val Gly Ile Phe Arg Ala Ala Val Cys Ser
145                 150                 155                 160

Arg Gly Val Ala Lys Ser Ile Asp Phe Ile Pro Val Glu Thr Leu Asp
                165                 170                 175

Ile Val Thr Arg Ser
            180
```

<210> 12
<211> 181
<212> PRT
<213> Hepatitis C virus

<400> 12

```
Ala Pro Ile Thr Ala Tyr Thr Gln Gln Thr Arg Gly Leu Leu Gly Ala
1               5                   10                  15

Ile Val Val Ser Leu Thr Gly Arg Asp Lys Asn Glu Gln Ala Gly Gln
            20                  25                  30

Val Gln Val Leu Ser Ser Val Thr Gln Thr Phe Leu Gly Thr Thr Ile
```

35 40 45

Ser Gly Val Leu Trp Thr Val Tyr His Gly Ala Gly Asn Lys Thr Leu
50 55 60

Ala Gly Pro Lys Gly Pro Val Thr Gln Met Tyr Thr Ser Ala Glu Gly
65 70 75 80

Asp Leu Val Gly Trp Pro Ser Pro Pro Gly Thr Lys Ser Leu Asp Pro
85 90 95

Cys Thr Cys Gly Ala Val Asp Leu Tyr Leu Val Thr Arg Asn Ala Asp
100 105 110

Val Ile Pro Val Arg Arg Lys Asp Asp Arg Arg Gly Ala Leu Leu Ser
115 120 125

Pro Arg Pro Leu Ser Thr Leu Lys Gly Ser Ser Gly Gly Pro Val Leu
130 135 140

Cys Ser Arg Gly His Ala Val Gly Leu Phe Arg Ala Ala Val Cys Ala
145 150 155 160

Arg Gly Val Ala Lys Ser Ile Asp Phe Ile Pro Val Glu Thr Leu Asp
165 170 175

Val Ala Thr Arg Thr
180

<210> 13
<211> 181
<212> PRT
<213> Hepatitis C virus

<400> 13

Ala Pro Ile Thr Ala Tyr Thr Gln Gln Thr Arg Gly Leu Leu Gly Ala
1 5 10 15

Ile Val Val Ser Leu Thr Gly Arg Asp Lys Asn Glu Gln Ala Gly Gln
20 25 30

Val Gln Val Leu Ser Ser Val Thr Gln Ser Phe Leu Gly Thr Thr Ile
35 40 45

Ser Gly Val Leu Trp Thr Val Tyr His Gly Ala Gly Asn Lys Thr Leu
50 55 60

Ala Gly Pro Lys Gly Pro Val Thr Gln Met Tyr Thr Ser Ala Glu Gly
65 70 75 80

Asp Leu Val Gly Trp Pro Ser Pro Pro Gly Thr Lys Ser Leu Asp Pro
85 90 95

```
Cys Thr Cys Gly Ala Val Asp Leu Tyr Leu Val Thr Arg Asn Ala Asp
            100                 105                 110

Val Ile Pro Val Arg Arg Lys Asp Asp Arg Arg Gly Ala Leu Leu Ser
        115                 120                 125

Pro Arg Pro Leu Ser Thr Leu Lys Gly Ser Ser Gly Gly Pro Val Leu
    130                 135                 140

Cys Ser Arg Gly His Ala Val Gly Leu Phe Arg Ala Ala Val Cys Ala
145                 150                 155                 160

Arg Gly Val Ala Lys Ser Ile Asp Phe Ile Pro Val Glu Thr Leu Asn
                165                 170                 175

Ile Ala Thr Arg Thr
            180

<210>  14
<211>  181
<212>  PRT
<213>  Hepatitis C virus

<400>  14

Ala Pro Ile Thr Ala Tyr Thr Gln Gln Thr Arg Gly Leu Leu Gly Ala
1               5                   10                  15

Ile Val Val Ser Leu Thr Gly Arg Asp Lys Asn Glu Gln Ala Gly Gln
            20                  25                  30

Val Gln Val Leu Ser Ser Val Thr Gln Ser Phe Leu Gly Thr Thr Ile
        35                  40                  45

Ser Gly Val Leu Trp Thr Val Tyr His Gly Ala Gly Asn Lys Thr Leu
    50                  55                  60

Ala Ser Pro Arg Gly Pro Val Thr Gln Met Tyr Thr Ser Ala Glu Gly
65                  70                  75                  80

Asp Leu Val Gly Trp Pro Ser Pro Pro Gly Thr Lys Ser Leu Asp Pro
                85                  90                  95

Cys Thr Cys Gly Ala Val Asp Leu Tyr Leu Val Thr Arg Asn Ala Asp
            100                 105                 110

Val Ile Pro Val Arg Arg Lys Asp Asp Arg Arg Gly Ala Leu Leu Ser
        115                 120                 125

Pro Arg Pro Leu Ser Thr Leu Lys Gly Ser Ser Gly Gly Pro Val Leu
    130                 135                 140

Cys Pro Arg Gly His Ala Val Gly Leu Phe Arg Ala Ala Val Cys Ala
```

145 150 155 160

Arg Gly Val Ala Lys Ser Ile Asp Phe Ile Pro Val Glu Ser Leu Asp
165 170 175

Ile Ala Arg Arg Thr
180

<210> 15
<211> 543
<212> DNA
<213> Hepatitis C virus

<400> 15
gccccgatca cagcatacgc ccagcaaact aggggccttc ttgggactat tgtgactagc 60

ttgactggca gggataagaa cgtggtgacc ggtgaagtgc aggtgctttc tacggctacc 120

cagaccttcc taggtacaac agtggggggg gttatgtgga ctgtttacca tggtgctggt 180

tcgagaacac tcgcgggcgc caaacatccc gcgctccaaa tgtacacaaa tgtagatcag 240

gacctcgttg ggtggccagc ccctccaggg gctaagtctc ttgaaccgtg cgcctgcggg 300

tctgcagact tatacttggt tacccgcgat gccgatgtca tccctgctcg gcgcaggggg 360

gactccacag cgagcttgct cagtcctagg cctctcgcct gtctcaaagg ttcctctgga 420

ggtcctgtta tgtgcccttc ggggcatgtt gcggggatct ttagggctgc tgtgtgcacc 480

agaggtgtag caaaagccct acagttcata ccagtggaaa cccttagtac acaggctagg 540

tct 543

<210> 16
<211> 181
<212> PRT
<213> Hepatitis C virus

<400> 16

Ala Pro Ile Thr Ala Tyr Ala Gln Gln Thr Arg Gly Leu Leu Gly Thr
1 5 10 15

Ile Val Thr Ser Leu Thr Gly Arg Asp Lys Asn Val Val Thr Gly Glu
20 25 30

Val Gln Val Leu Ser Thr Ala Thr Gln Thr Phe Leu Gly Thr Thr Val
35 40 45

Gly Gly Val Met Trp Thr Val Tyr His Gly Ala Gly Ser Arg Thr Leu
50 55 60

Ala Gly Ala Lys His Pro Ala Leu Gln Met Tyr Thr Asn Val Asp Gln
65 70 75 80

Asp Leu Val Gly Trp Pro Ala Pro Pro Gly Ala Lys Ser Leu Glu Pro
85 90 95

```
Cys Ala Cys Gly Ser Ala Asp Leu Tyr Leu Val Thr Arg Asp Ala Asp
            100                 105                 110

Val Ile Pro Ala Arg Arg Arg Gly Asp Ser Thr Ala Ser Leu Leu Ser
        115                 120                 125

Pro Arg Pro Leu Ala Cys Leu Lys Gly Ser Ser Gly Gly Pro Val Met
    130                 135                 140

Cys Pro Ser Gly His Val Ala Gly Ile Phe Arg Ala Ala Val Cys Thr
145                 150                 155                 160

Arg Gly Val Ala Lys Ala Leu Gln Phe Ile Pro Val Glu Thr Leu Ser
                165                 170                 175

Thr Gln Ala Arg Ser
            180

<210>  17
<211>  181
<212>  PRT
<213>  Hepatitis C virus

<400>  17

Ala Pro Ile Thr Ala Tyr Ala Gln Gln Thr Arg Gly Leu Leu Gly Thr
1               5                   10                  15

Ile Val Thr Ser Leu Thr Gly Arg Asp Lys Asn Val Val Thr Gly Glu
            20                  25                  30

Val Gln Val Leu Ser Thr Ala Thr Gln Thr Phe Leu Gly Thr Thr Val
        35                  40                  45

Gly Gly Val Thr Trp Thr Val Tyr His Gly Ala Gly Ser Arg Thr Leu
    50                  55                  60

Ala Gly Ala Lys His Pro Ala Leu Gln Met Tyr Thr Asn Val Asp Gln
65                  70                  75                  80

Asp Leu Val Gly Trp Pro Ala Pro Pro Gly Ala Lys Ser Leu Glu Pro
                85                  90                  95

Cys Ala Cys Gly Ser Ala Asp Leu Tyr Leu Val Thr Arg Asp Ala Asp
            100                 105                 110

Val Ile Pro Ala Arg Arg Arg Gly Asp Ser Thr Ala Ser Leu Leu Ser
        115                 120                 125

Pro Arg Pro Leu Ala Cys Leu Lys Gly Ser Ser Gly Gly Pro Val Met
    130                 135                 140

Cys Pro Ser Gly His Val Ala Gly Ile Phe Arg Ala Ala Val Cys Thr
```

145 150 155 160

Arg Gly Val Ala Lys Ala Leu Gln Phe Ile Pro Val Glu Thr Leu Ser
165 170 175

Thr Gln Thr Arg Ser
180

<210> 18
<211> 181
<212> PRT
<213> Hepatitis C virus

<400> 18

Ala Pro Ile Thr Ala Tyr Ala Gln Gln Thr Arg Gly Leu Leu Gly Thr
1 5 10 15

Ile Val Thr Ser Leu Thr Gly Arg Asp Lys Asn Val Val Thr Gly Glu
20 25 30

Val Gln Val Leu Ser Thr Ala Thr Gln Thr Phe Leu Gly Thr Thr Val
35 40 45

Gly Gly Val Ile Trp Thr Val Tyr His Gly Ala Gly Ser Arg Thr Leu
50 55 60

Ala Gly Ala Lys His Pro Ala Leu Gln Met Tyr Thr Asn Val Asp Gln
65 70 75 80

Asp Leu Val Gly Trp Pro Ala Pro Pro Gly Ala Lys Ser Leu Glu Pro
85 90 95

Cys Ala Cys Gly Ser Ser Asp Leu Tyr Leu Val Thr Arg Asp Ala Asp
100 105 110

Val Ile Pro Ala Arg Arg Arg Gly Asp Ser Thr Ala Ser Leu Leu Ser
115 120 125

Pro Arg Pro Leu Ala Cys Leu Lys Gly Ser Ser Gly Gly Pro Val Met
130 135 140

Cys Pro Ser Gly His Val Ala Gly Ile Phe Arg Ala Ala Val Cys Thr
145 150 155 160

Arg Gly Val Ala Lys Ser Leu Gln Phe Ile Pro Val Glu Thr Leu Ser
165 170 175

Thr Gln Ala Arg Ser
180

<210> 19
<211> 181
<212> PRT

<213> Hepatitis C virus

<400> 19

```
Ala Pro Ile Thr Ala Tyr Ala Gln Gln Thr Arg Gly Leu Leu Gly Thr
1               5                   10                  15

Ile Val Thr Ser Leu Thr Gly Arg Asp Lys Asn Val Val Ala Gly Glu
            20                  25                  30

Val Gln Val Leu Ser Thr Ala Thr Gln Thr Phe Leu Gly Thr Thr Val
        35                  40                  45

Gly Gly Val Met Trp Thr Val Tyr His Gly Ala Gly Ser Arg Thr Leu
    50                  55                  60

Ala Gly Val Lys His Pro Ala Leu Gln Met Tyr Thr Asn Val Asp Gln
65                  70                  75                  80

Asp Leu Val Gly Trp Pro Ala Pro Pro Gly Ala Lys Ser Leu Glu Pro
                85                  90                  95

Cys Thr Cys Gly Ser Ala Asp Leu Tyr Leu Val Thr Arg Asp Ala Asp
            100                 105                 110

Val Ile Pro Ala Arg Arg Arg Gly Asp Ser Thr Ala Ser Leu Leu Ser
        115                 120                 125

Pro Arg Pro Leu Ala Arg Leu Lys Gly Ser Ser Gly Gly Pro Val Met
    130                 135                 140

Cys Pro Ser Gly His Val Ala Gly Ile Phe Arg Ala Ala Val Cys Thr
145                 150                 155                 160

Arg Gly Val Ala Lys Ala Leu Gln Phe Ile Pro Val Glu Thr Leu Ser
                165                 170                 175

Thr Gln Ala Arg Ser
            180
```

<210> 20
<211> 181
<212> PRT
<213> Hepatitis C virus

<400> 20

```
Ala Pro Ile Thr Ala Tyr Ala Gln Gln Thr Arg Gly Leu Leu Gly Thr
1               5                   10                  15

Ile Val Thr Gly Leu Thr Gly Arg Asp Lys Asn Val Val Thr Gly Glu
            20                  25                  30

Val Gln Val Leu Ser Thr Ala Thr Gln Thr Phe Leu Gly Thr Thr Val
        35                  40                  45
```

```
Gly Gly Val Met Trp Thr Val Tyr His Gly Ala Gly Ser Arg Thr Leu
    50                  55                  60

Ala Gly Ala Lys His Pro Ala Leu Gln Met Tyr Thr Asn Val Asp Gln
65                  70                  75                  80

Asp Leu Val Gly Trp Pro Ala Pro Pro Gly Ala Lys Ser Leu Glu Pro
                85                  90                  95

Cys Ala Cys Gly Ser Ala Asp Leu Tyr Leu Val Thr Arg Asp Ala Asp
            100                 105                 110

Val Ile Pro Ala Arg Arg Arg Gly Asp Ser Thr Ala Ser Leu Leu Ser
        115                 120                 125

Pro Arg Pro Leu Ala Cys Leu Lys Gly Ser Ser Gly Gly Pro Val Met
    130                 135                 140

Cys Pro Ser Gly His Val Ala Gly Ile Phe Arg Ala Ala Val Cys Thr
145                 150                 155                 160

Arg Gly Val Ala Lys Ala Leu Gln Phe Ile Pro Val Glu Thr Leu Ser
                165                 170                 175

Thr Gln Ala Arg Ser
            180

<210>  21
<211>  181
<212>  PRT
<213>  Hepatitis C virus

<400>  21

Ser Pro Ile Ser Ala Tyr Ala Gln Gln Thr Arg Gly Leu Phe Gly Thr
1               5                   10                  15

Ile Val Thr Ser Leu Thr Gly Arg Asp Lys Asn Val Val Thr Gly Glu
            20                  25                  30

Val Gln Val Leu Ser Thr Ala Thr Gln Thr Phe Leu Gly Thr Thr Val
        35                  40                  45

Gly Gly Val Met Trp Thr Val Tyr His Gly Ala Gly Ser Arg Thr Leu
    50                  55                  60

Ala Gly Asn Lys Arg Pro Ala Leu Gln Met Tyr Thr Asn Val Asp Gln
65                  70                  75                  80

Asp Leu Val Gly Trp Pro Ala Pro Ala Gly Thr Lys Ser Leu Asp Pro
                85                  90                  95
```

```
Cys Thr Cys Gly Ser Ser Asp Leu Tyr Leu Val Thr Arg Glu Ala Asp
            100                 105                 110

Val Leu Pro Ala Arg Arg Arg Gly Asp Ser Thr Ala Ser Leu Leu Ser
        115                 120                 125

Thr Arg Pro Leu Ser Cys Leu Lys Gly Ser Ser Gly Gly Pro Val Met
    130                 135                 140

Cys Pro Ser Gly His Val Val Gly Ile Phe Arg Ala Ala Val Cys Thr
145                 150                 155                 160

Arg Gly Val Ala Lys Ala Leu Gln Phe Ile Pro Val Glu Thr Leu Ser
                165                 170                 175

Thr Gln Val Arg Ser
            180


<210>  22
<211>  181
<212>  PRT
<213>  Hepatitis C virus

<400>  22

Ala Pro Ile Thr Ala His Ala Gln Gln Thr Arg Gly Leu Phe Gly Thr
1               5                   10                  15

Ile Val Thr Ser Leu Thr Gly Arg Asp Lys Asn Ile Val Thr Gly Glu
            20                  25                  30

Ile Gln Val Leu Ser Thr Ser Thr Gln Thr Phe Leu Gly Thr Ser Val
        35                  40                  45

Gly Gly Val Met Trp Thr Val Tyr His Gly Ala Gly Ser Arg Thr Leu
    50                  55                  60

Ala Gly Asn Lys Arg Pro Ala Leu Gln Met Tyr Thr Asn Val Asp Gln
65                  70                  75                  80

Asp Leu Val Gly Trp Pro Ser Pro Pro Gly Ala Lys Ser Leu Val Pro
                85                  90                  95

Cys Thr Cys Gly Ser Ala Asp Leu Tyr Leu Ile Thr Arg Asp Ala Asp
            100                 105                 110

Val Leu Pro Ala Arg Arg Arg Gly Asp Ser Thr Ala Ser Leu Leu Ser
        115                 120                 125

Pro Arg Pro Leu Ala Cys Leu Lys Gly Ser Ser Gly Gly Pro Ile Met
    130                 135                 140

Cys Pro Ser Gly His Val Ala Gly Ile Phe Arg Ala Ala Val Cys Thr
```

145 150 155 160

Arg Gly Val Ala Lys Ala Leu Gln Phe Ile Pro Val Glu Ser Leu Ser
165 170 175

Ala Gln Thr Arg Ser
180

<210> 23
<211> 181
<212> PRT
<213> Hepatitis C virus

<400> 23

Ala Pro Ile Thr Ala Tyr Ala Gln Gln Thr Arg Gly Leu Leu Gly Cys
1 5 10 15

Ile Ile Thr Ser Leu Thr Gly Arg Asp Lys Asn Gln Val Glu Gly Glu
20 25 30

Val Gln Ile Val Ser Thr Ala Thr Gln Thr Phe Leu Ala Thr Cys Ile
35 40 45

Asn Gly Val Cys Trp Thr Val Tyr His Gly Ala Gly Thr Arg Thr Ile
50 55 60

Ala Ser Pro Lys Gly Pro Val Ile Gln Met Tyr Thr Asn Val Asp Gln
65 70 75 80

Asp Leu Val Gly Trp Pro Ala Pro Gln Gly Ser Arg Ser Leu Thr Pro
85 90 95

Cys Thr Cys Gly Ser Ser Asp Leu Tyr Leu Val Thr Arg His Ala Asp
100 105 110

Val Ile Pro Val Arg Arg Arg Gly Asp Ser Arg Gly Ser Leu Leu Ser
115 120 125

Pro Arg Pro Ile Ser Tyr Leu Lys Gly Ser Ser Gly Gly Pro Leu Leu
130 135 140

Cys Pro Ala Gly His Ala Val Gly Leu Phe Arg Ala Ala Val Cys Thr
145 150 155 160

Arg Gly Val Thr Lys Ala Val Asp Phe Ile Pro Val Glu Asn Leu Glu
165 170 175

Thr Thr Met Arg Ser
180

<210> 24
<211> 181
<212> PRT

<213> Hepatitis C virus

<400> 24

Ala Pro Ile Thr Ala Tyr Ala Gln Gln Thr Arg Gly Leu Leu Gly Cys
1 5 10 15

Ile Ile Thr Ser Leu Thr Gly Arg Asp Lys Asn Gln Val Glu Gly Glu
20 25 30

Val Gln Val Val Ser Thr Ala Thr Gln Ser Phe Leu Ala Thr Cys Ile
35 40 45

Asn Gly Val Cys Trp Thr Val Tyr His Gly Ala Gly Ser Lys Thr Leu
50 55 60

Ala Gly Pro Lys Gly Pro Ile Thr Gln Met Tyr Thr Asn Val Asp Gln
65 70 75 80

Asp Leu Val Gly Trp Pro Ala Pro Pro Gly Ala Arg Ser Leu Thr Pro
85 90 95

Cys Thr Cys Gly Ser Ser Asp Leu Tyr Leu Val Thr Arg His Ala Asp
100 105 110

Val Ile Pro Val Arg Arg Arg Gly Asp Ser Arg Gly Ser Leu Leu Ser
115 120 125

Pro Arg Pro Ile Ser Tyr Leu Lys Gly Ser Ser Gly Gly Pro Leu Leu
130 135 140

Cys Pro Ser Gly His Ala Val Gly Ile Phe Arg Ala Ala Val Cys Thr
145 150 155 160

Arg Gly Val Ala Lys Ala Val Asp Phe Ile Pro Val Glu Ser Met Glu
165 170 175

Thr Thr Met Arg Ser
180

<210> 25
<211> 181
<212> PRT
<213> Hepatitis C virus

<400> 25

Ala Pro Ile Thr Ala Tyr Ser Gln Gln Thr Arg Gly Leu Leu Gly Cys
1 5 10 15

Ile Ile Thr Ser Leu Thr Gly Arg Asp Lys Asn Gln Val Glu Gly Glu
20 25 30

Val Gln Val Val Ser Thr Ala Thr Gln Ser Phe Leu Ala Thr Cys Val
35 40 45

Asn Gly Val Cys Trp Thr Val Phe His Gly Ala Gly Ser Lys Thr Leu
50 55 60

Ala Gly Pro Lys Gly Pro Ile Thr Gln Met Tyr Thr Asn Val Asp Gln
65 70 75 80

Asp Leu Val Gly Trp Gln Ala Pro Pro Gly Ala Arg Ser Met Thr Pro
85 90 95

Cys Thr Cys Gly Ser Ser Asp Leu Tyr Leu Val Thr Arg His Ala Asp
100 105 110

Val Ile Pro Val Arg Arg Arg Gly Asp Gly Arg Gly Ser Leu Leu Ser
115 120 125

Pro Arg Pro Val Ser Tyr Leu Lys Gly Ser Ser Gly Gly Pro Leu Leu
130 135 140

Cys Pro Ser Gly His Val Val Gly Val Phe Arg Ala Ala Val Cys Thr
145 150 155 160

Arg Gly Val Ala Lys Ala Val Asp Phe Val Pro Val Glu Ser Met Glu
165 170 175

Thr Thr Met Arg Ser
180

<210> 26
<211> 181
<212> PRT
<213> Hepatitis C virus

<400> 26

Ala Pro Ile Thr Ala Tyr Ala Gln Gln Thr Arg Gly Leu Leu Gly Ala
1 5 10 15

Ile Val Val Ser Met Thr Gly Arg Asp Lys Thr Glu Gln Ala Gly Glu
20 25 30

Ile Gln Val Leu Ser Thr Val Thr Gln Ser Phe Leu Gly Thr Thr Ile
35 40 45

Ser Gly Val Leu Trp Thr Val Tyr His Gly Ala Gly Asn Lys Thr Leu
50 55 60

Ala Gly Ser Arg Gly Pro Val Thr Gln Met Tyr Ser Ser Ala Glu Gly
65 70 75 80

Asp Leu Val Gly Trp Pro Ser Pro Pro Gly Thr Lys Ser Leu Glu Pro
85 90 95

Cys Thr Cys Gly Ala Val Asp Leu Tyr Leu Val Thr Arg Asn Ala Asp
100 105 110

Val Ile Pro Ala Arg Arg Arg Gly Asp Lys Arg Gly Ala Leu Leu Ser
115 120 125

Pro Arg Pro Leu Ser Thr Leu Lys Gly Ser Ser Gly Gly Pro Val Leu
130 135 140

Cys Pro Arg Gly His Ala Val Gly Ile Phe Arg Ala Ala Val Cys Ser
145 150 155 160

Arg Gly Val Ala Lys Ser Ile Asp Phe Ile Pro Val Glu Thr Leu Asp
165 170 175

Ile Val Thr Arg Ser
180

<210> 27
<211> 181
<212> PRT
<213> Hepatitis C virus

<400> 27

Ala Pro Ile Thr Ala Tyr Thr Gln Gln Thr Arg Gly Leu Leu Gly Ala
1 5 10 15

Ile Val Val Ser Leu Thr Gly Arg Asp Lys Asn Glu Gln Ala Gly Gln
20 25 30

Val Gln Val Leu Ser Ser Val Thr Gln Ser Phe Leu Gly Thr Ser Ile
35 40 45

Ser Gly Val Leu Trp Thr Val Tyr His Gly Ala Gly Asn Lys Thr Leu
50 55 60

Ala Gly Pro Lys Gly Pro Val Thr Gln Met Tyr Thr Ser Ala Glu Gly
65 70 75 80

Asp Leu Val Gly Trp Pro Ser Pro Pro Gly Thr Lys Ser Leu Asp Pro
85 90 95

Cys Thr Cys Gly Ala Val Asp Leu Tyr Leu Val Thr Arg Asn Ala Asp
100 105 110

Val Ile Pro Val Arg Arg Lys Asp Asp Arg Arg Gly Ala Leu Leu Ser
115 120 125

Pro Arg Pro Leu Ser Thr Leu Lys Gly Ser Ser Gly Gly Pro Val Leu
130 135 140

Cys Ser Arg Gly His Ala Val Gly Leu Phe Arg Ala Ala Val Cys Ala
145 150 155 160

```
Arg Gly Val Ala Lys Ser Ile Asp Phe Ile Pro Val Glu Ser Leu Asp
                165                 170                 175

Ile Ala Thr Arg Thr
            180

<210>  28
<211>  181
<212>  PRT
<213>  Hepatitis C virus

<400>  28

Ala Pro Ile Thr Ala Tyr Ala Gln Gln Thr Arg Gly Leu Leu Gly Thr
1               5                   10                  15

Ile Val Thr Ser Leu Thr Gly Arg Asp Lys Asn Val Val Thr Gly Glu
            20                  25                  30

Val Gln Val Leu Ser Thr Ala Thr Gln Thr Phe Leu Gly Thr Thr Val
        35                  40                  45

Gly Gly Val Met Trp Thr Val Tyr His Gly Ala Gly Ser Arg Thr Leu
    50                  55                  60

Ala Gly Ala Lys His Pro Ala Leu Gln Met Tyr Thr Asn Val Asp Gln
65                  70                  75                  80

Asp Leu Val Gly Trp Pro Ala Pro Pro Gly Ala Lys Ser Leu Glu Pro
                85                  90                  95

Cys Ala Cys Gly Ser Ala Asp Leu Tyr Leu Val Thr Arg Asp Ala Asp
            100                 105                 110

Val Ile Pro Ala Arg Arg Arg Gly Asp Ser Thr Ala Ser Leu Leu Ser
        115                 120                 125

Pro Arg Pro Leu Ala Cys Leu Lys Gly Ser Ser Gly Gly Pro Val Met
    130                 135                 140

Cys Pro Ser Gly His Val Ala Gly Ile Phe Arg Ala Ala Val Cys Thr
145                 150                 155                 160

Arg Gly Val Ala Lys Ala Leu Gln Phe Ile Pro Val Glu Thr Leu Ser
                165                 170                 175

Thr Gln Ala Arg Ser
            180

<210>  29
<211>  181
<212>  PRT
<213>  Hepatitis C virus
```

<220>
<221> misc_feature
<222> (179)..(179)
<223> Xaa can be any naturally occurring amino acid

<400> 29

Ala Pro Ile Thr Ala Tyr Ala Gln Gln Thr Arg Gly Leu Phe Gly Thr
1 5 10 15

Ile Val Thr Ser Leu Thr Gly Arg Asp Lys Asn Val Val Thr Gly Glu
20 25 30

Val Gln Val Leu Ser Thr Ala Thr Gln Thr Phe Leu Gly Thr Thr Val
35 40 45

Gly Gly Val Met Trp Thr Val Tyr His Gly Ala Gly Ser Arg Thr Leu
50 55 60

Ala Gly Asn Lys Arg Pro Ala Leu Gln Met Tyr Thr Asn Val Asp Gln
65 70 75 80

Asp Leu Val Gly Trp Pro Ala Pro Pro Gly Ala Lys Ser Leu Asp Pro
85 90 95

Cys Thr Cys Gly Ser Ala Asp Leu Tyr Leu Val Thr Arg Asp Ala Asp
100 105 110

Val Leu Pro Ala Arg Arg Arg Gly Asp Ser Thr Ala Ser Leu Leu Ser
115 120 125

Pro Arg Pro Leu Ala Cys Leu Lys Gly Ser Ser Gly Gly Pro Val Met
130 135 140

Cys Pro Ser Gly His Val Ala Gly Ile Phe Arg Ala Ala Val Cys Thr
145 150 155 160

Arg Gly Val Ala Lys Ala Leu Gln Phe Ile Pro Val Glu Thr Leu Ser
165 170 175

Thr Gln Xaa Arg Ser
180

<210> 30
<211> 9
<212> PRT
<213> Hepatitis C virus

<400> 30

Glu Asp Val Val Cys Ser Met Ser Tyr
1 5

<210> 31
<211> 16
<212> PRT
<213> Hepatitis C virus

<400> 31

```
Lys Lys Gly Ser Val Val Ile Val Gly Arg Ile Val Leu Ser Gly Lys
1               5                   10                  15
```

<210> 32
<211> 17
<212> PRT
<213> Hepatitis C virus

<400> 32

```
Lys Lys Gly Ser Val Ser Ile Ile Gly Arg Leu His Ile Asn Gln Arg
1               5                   10                  15


Ala
```

<210> 33
<211> 17
<212> PRT
<213> Hepatitis C virus

<400> 33

```
Lys Lys Gly Ser Val Val Ile Val Gly His Ile Glu Leu Gly Gly Lys
1               5                   10                  15


Pro
```

## Description

TECHNICAL FIELD OF THE INVENTION

The present invention relates to compounds that inhibit serine protease activity, particularly the activity of hepatitis C virus NS3-NS4A protease. As such, they act by interfering with the life cycle of the hepatitis C virus and are also useful as antiviral agents. The invention further relates to compositions for either ex vivo use or for administration to a patient suffering from HCV infection. The invention also relates to methods of treating an HCV infection in a patient by administering a composition of this invention.

BACKGROUND OF THE INVENTION

Infection by hepatitis C virus ("HCV") is a compelling human medical problem. HCV is recognized as the causative agent for most cases of non-A, non-B hepatitis, with an estimated human sero-prevalence of 3% globally [A. Alberti et al., "Natural History of Hepatitis C," J. Hepatology, 31., (Suppl. 1), pp. 17-24 (1999)]. Nearly four million individuals may be infected in the United States alone [M.J. Alter et al., "The Epidemiology of Viral Hepatitis in the United States, Gastroenterol. Clin. North Am., 23, pp. 437-455 (1994); M. J. Alter "Hepatitis C Virus Infection in the United States," J. Hepatology, 31., (Suppl. 1), pp. 88-91 (1999)].

Upon first exposure to HCV only about 20% of infected individuals develop acute clinical hepatitis while others appear to resolve the infection spontaneously. In almost 70% of instances, however, the virus establishes a chronic infection that persists for decades [S. Iwarson, "The Natural Course of Chronic Hepatitis," FEMS Microbiology Reviews, 14, pp. 201-204 (1994); D. Lavanchy, "Global Surveillance and Control of Hepatitis C," J. Viral Hepatitis, 6, pp. 35-47 (1999)]. This usually results in recurrent and progressively worsening liver inflammation, which often leads to more severe disease states such as cirrhosis and hepatocellular carcinoma [M.C. Kew, "Hepatitis C and Hepatocellular Carcinoma", FEMS Microbiology Reviews, 14, pp. 211-220 (1994); I. Saito et al., "Hepatitis C Virus Infection is Associated with the Development of Hepatocellular Carcinoma," Proc. Natl. Acad. Sci. USA, 87, pp. 6547-6549 (1990)]. Unfortunately, there are no broadly effective treatments for the debilitating progression of chronic HCV.

The HCV genome encodes a polyprotein of 3010-3033 amino acids [Q.L. Choo, et al., "Genetic Organization and Diversity of the Hepatitis C Virus." Proc. Natl. Acad. Sci. USA, 88, pp. 2451-2455 (1991); N. Kato et al., "Molecular Cloning of the Human Hepatitis C Virus Genome From Japanese Patients with Non-A, Non-B Hepatitis," Proc. Natl. Acad. Sci. USA, 87, pp. 9524-9528 (1990); A. Takamizawa et al., "Structure and Organization of the Hepatitis C Virus Genome Isolated From Human Carriers," J. Virol., 65, pp. 1105-1113 (1991)]. The HCV nonstructural (NS) proteins are presumed to provide the essential catalytic machinery for viral replication. The NS proteins are derived by proteolytic cleavage of the polyprotein [R. Bartenschlager et al., "Nonstructural Protein 3 of the Hepatitis C Virus Encodes a Serine-Type Proteinase Required for Cleavage at the NS3/4 and NS4/5 Junctions," J. Virol., 67, pp. 3835-3844 (1993); A. Grakoui et al., "Characterization of the Hepatitis C Virus-Encoded Serine Proteinase: Determination of Proteinase-Dependent Polyprotein Cleavage Sites," J. Virol., 67, pp. 2832-2843 (1993); A. Grakoui et al., "Expression and Identification of Hepatitis C Virus Polyprotein Cleavage Products," J. Virol., 67, pp. 1385-1395 (1993); L. Tomei et al., "NS3 is a serine protease required for processing of hepatitis C virus polyprotein", J. Virol., 67, pp. 4017-4026 (1993)].

The HCV NS protein 3 (NS3) contains a serine protease activity that helps process the majority of the viral enzymes, and is thus considered essential for viral replication and infectivity. The HCV NS3 serine protease is essential for viral replication since the substitutions of the catalytic triad resulted in loss of infectivity in chimpanzees [A.A. Kolykhalov et al., "Hepatitis C virus-encoded enzymatic activities and conserved RNA elements in the 3' nontranslated region are essential for virus replication in vivo", J. Virol., 74: 2046-2051]. The first 181 amino acids of NS3 (residues 1027-1207 of the viral polyprotein) have been shown to contain the serine protease domain of NS3 that processes all four downstream sites of the HCV polyprotein [C. Lin et al., "Hepatitis C Virus NS3 Serine Proteinase: Trans-Cleavage Requirements and Processing Kinetics", J. Virol., 68, pp. 8147-8157 (1994)].

The HCV NS3 serine protease and its associated cofactor, NS4A, helps process the viral non-structural protein region into individual non-structural proteins, including all of the viral enzymes. This processing appears to be analogous to that carried out by the human immunodeficiency virus aspartyl protease, which is also involved in processing of viral proteins. HIV protease inhibitors, which inhibit viral protein processing are potent antiviral agents in man, indicating that interrupting this stage of the viral life cycle results in therapeutically active agents. Consequently it is an attractive target for drug discovery.

Several potential HCV protease inhibitors have been described in the prior art [PCT publication Nos. WO 02/18369, WO 02/08244, WO 00/09558, WO 00/09543, WO 99/64442, WO 99/07733, WO 99/07734, WO 99/50230, WO 98/46630, WO 98/17679 and WO 97/43310, United States Patent 5,990,276, M. Llinas-Brunet et al., Bioorg. Med. Chem. Lett., 8, pp. 1713-18 (1998); W. Han et al., Bioorg. Med. Chem. Lett., 10, 711-13 (2000); R. Dunsdon et al., Bioorg. Med. Chem. Lett., 10, pp. 1571-79 (2000); M. Llinas-Brunet et al., Bioorg. Med. Chem. Lett., 10, pp. 2267-70

(2000); and S. LaPlante et al., Bioorg. Med. Chem. Lett., 10, pp. 2271-74 (2000)]. It is not known however whether these compounds would have the appropriate profiles to be acceptable drugs. Furthermore, most, if not all of these inhibitors were discovered using the genotype 1 (1a or 1b) NS3-4A serine protease as the target. However, there are a variety of genotypes of HCV, and a variety of subtypes within each genotype. For example, at present it is known that there are eleven (numbered 1 through 11) main genotypes of HCV, although others have classified the genotypes as 6 main genotypes. Each of these genotypes is further subdivided into subtypes (1a -1c; 2a-2c; 3a-3b; 4a-4e; 5a; 6a; 7a-7b; 8a-8b; 9a; 10a; and 11a).

The prevalence of the subtypes varies globally as follows:

| | |
|---|---|
| 1a | Found mostly in North and South America; common in Australia |
| 1b | Found mostly in Europe and Asia |
| 2a | Most common genotype 2 in Japan and China |
| 2b | Most common genotype 2 in US and Northern Europe |
| 2c | Most common genotype 2 in Western and Southern Europe |
| 3a | Highly prevalent in Australia and South Asia |
| 4a | Highly prevalent in Egypt |
| 4c | Highly prevalent in Central Africa |
| 5a | Highly prevalent in South Africa |
| 6a | Restricted to Hong Kong, Macau and Vietnam |
| 7a & 7b | Common in Thailand |
| 8a, 8b & 8c | Prevalent in Vietnam |
| 10a and 11a | Found in Indonesia |

The current scientific belief is that HCV genotype or subtype may determine the responsiveness of the patient to therapy. While it has been noted that there is a correlation between the degree of genomic complexity of the HCV and the patient's response to interferon therapy the reason for this correlation is unclear. It is generally accepted that genotype 2 HCV and genotype 3 HCV virus-infected patients respond to conventional therapy to a different degree than those patient infected with genotype 1 HCV. Thus, while a number of HCV protease inhibitors have been designed/ discovered against genotype 1 HCV protease, it is not clear whether these inhibitors will effectively inhibit the HCV NS3-4A serine proteases from other genotypes, such as for example genotype 2 HCV and genotype 3 HCV.

Therefore, the current understanding of HCV has not led to any satisfactory anti-HCV agents or treatments. The only established therapy for HCV disease is combination treatment of pegylated interferon plus ribavirin. However, interferons have significant side effects [M. A. Wlaker et al., "Hepatitis C Virus: An Overview of Current Approaches and Progress," DDT, 4, pp. 518-29 (1999); D. Moradpour et al., "Current and Evolving Therapies for Hepatitis C," Eur. J. Gastroenterol. Hepatol., 11, pp. 1199-1202 (1999); H. L. A. Janssen et al. "Suicide Associated with Alfa-Interferon Therapy for Chronic Viral Hepatitis," J. Hepatol., 21, pp..241-243 (1994); P.F. Renault et al., "Side Effects of Alpha Interferon," Seminars in Liver Disease, 9, pp. 273-277. (1989)] and induce long term remission in only a fraction (~ 25%) of cases [O. Weiland, "Interferon Therapy in Chronic Hepatitis C Virus Infection" , FEMS Microbiol. Rev., 14, pp. 279-288 (1994)]. In addition, this combination treatment has roughly 80% sustained viral response (SVR) for patients infected with genotype 2 or 3 HCV and 40-50% SVR in genotype 1 HCV-infected patients [J.G. McHutchison, et al., N. Engl. J. Med., 339: 1485-1492 (1998); G.L. Davis et al., N. Engl. J. Med., 339: 1493-1499 (1998)]. Moreover, the prospects for effective anti-HCV vaccines remain uncertain.

Thus, there is a need for more effective anti-HCV therapies, particularly compounds that inhibit HCV NS3 protease. Such compounds may be useful as antiviral agents, particularly as anti-HCV agents. There is also a need for compounds that inhibit various genotypes of the HCV serine protease.

## SUMMARY OF THE INVENTION

The present invention addresses these needs by providing a method for inhibiting genotype-2 and genotype-3 HCV with VX-950. While the present invention exemplifies that VX-950 is superior to other protease inhibitors at specifically inhibiting genotype-2 and genotype-3 HCV, it is contemplated that other non-genotype 1 HCV genotypes

also may be beneficially inhibited by VX-950.

The invention also relates to compositions that comprise the VX-950 and the use thereof. Such compositions may be used to pre-treat invasive devices to be inserted into a patient, to treat biological samples, such as blood, prior to administration to a patient, and for direct administration to a patient. In each case the composition will be used to inhibit HCV replication and to lessen the risk of or the severity of HCV infection.

BRIEF DESCRIPTION OF THE DRAWINGS

Fig. 1 The alignment of amino acid sequence of eleven genotype 2 HCV NS3 serine protease domains
Fig. 2. The consensus amino acid and nucleotide sequence of genotype 2a NS3 serine protease domain
Fig. 3. The alignment of amino acid sequence of six genotype 3 HCV NS3 serine protease domains.
Fig. 4 The consensus amino acid and nucleotide sequence of genotype 3a NS3 serine protease domain
Fig. 5. The alignment of the consensus amino acid sequence of each genotype or subgenotype 1a, 1b, 2a, 2b, 3a and 3b.

DETAILED DESCRIPTION OF THE INVENTION

The present invention provides methods for inhibiting genotype-2 and genotype-3 protease, either alone or together by contacting the genotype-2 or genotype-3 protease with VX-950.

VX-950 is a competitive, reversible peptidomimetic NS3/4A protease inhibitor with a steady state binding constant (ki*) of 3nM (and with a Ki of 8 nM) [WO 02/018369]. VX-950 may be prepared in general by methods known to those skilled in the art (see, e.g., WO 02/18369).

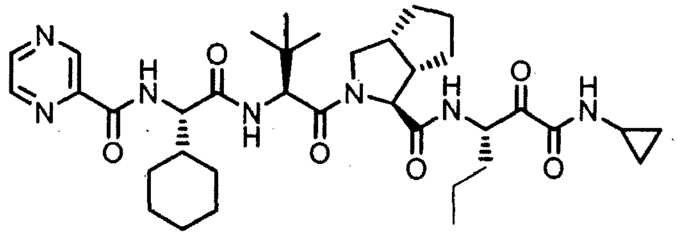

VX-950

A compound of this invention may contain one or more asymmetric carbon atoms and thus may occur as racemates and racemic mixtures, single enantiomers, diastereomeric mixtures and individual diastereomers. All such isomeric forms of these compounds are expressly included in the present invention. Each stereogenic carbon may be of the R or S configuration.

For example, in certain embodiments, compounds used may be mixtures of the D- and L-isomers at the N-propyl-side chain as depicted in the following structure:

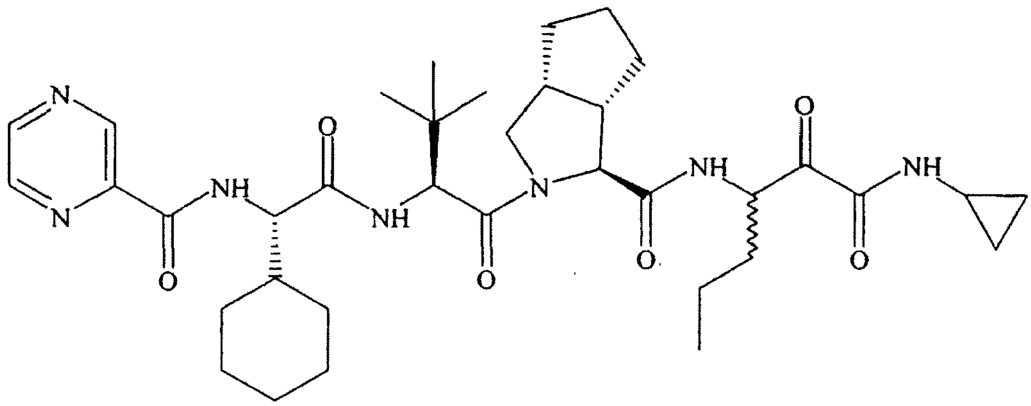

**Structure A**

Other agents generated through rational drug design using e.g., VX-950 or the compound of Structure A as a starting compound may be tested for their activity as protease inhibitors.

Preferably, the compounds of this invention have the structure and stereochemistry depicted in compounds in VX-950.

Another embodiment of this invention provides a composition comprising VX-950 or a pharmaceutically acceptable salt thereof. According to a preferred embodiment, VX-950 is present in an amount effective to decrease the viral load in a sample or in a patient, wherein said virus encodes a serine protease necessary for the viral life cycle, and a pharmaceutically acceptable carrier.

If pharmaceutically acceptable salts of a compound of this invention are utilized in these compositions, those salts are preferably derived from inorganic or organic acids and bases. Included among such acid salts are the following: acetate, adipate, alginate, aspartate, benzoate, benzene sulfonate, bisulfate, butyrate, citrate, camphorate, camphor sulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenyl-propionate, picrate, pivalate, propionate, succinate, tartrate, thiocyanate, tosylate and undecanoate. Base salts include ammonium salts, alkali metal salts, such as sodium and potassium salts, alkaline earth metal salts, such as calcium and magnesium salts, salts with organic bases, such as dicyclohexylamine salts, N-methyl-D-glucamine, and salts with amino acids such as arginine, lysine, and so forth.

Also, the basic nitrogen-containing groups may be quaternized with such agents as lower alkyl halides, such as methyl, ethyl, propyl, and butyl chloride, bromides and iodides; dialkyl sulfates, such as dimethyl, diethyl, dibutyl and diamyl sulfates, long chain halides such as decyl, lauryl, myristyl and stearyl chlorides, bromides and iodides, aralkyl halides, such as benzyl and phenethyl bromides and others. Water or oil-soluble or dispersible products are thereby obtained.

The compounds utilized in the compositions and methods of this invention may also be modified by appending appropriate functionalities to enhance selective biological properties. Such modifications are known in the art and include those which increase biological penetration into a given biological system (e.g., blood, lymphatic system, central nervous system), increase oral availability, increase solubility to allow administration by injection, alter metabolism and alter rate of excretion.

Pharmaceutically acceptable carriers that may be used in these compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

According to a preferred embodiment, the compositions of this invention are formulated for pharmaceutical administration to a mammal, preferably a human being.

Such pharmaceutical compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally or intravenously.

Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

Dosage levels of between about 0.01 and about 100 mg/kg body weight per day, preferably between about 0.5 and about 75 mg/kg body weight per day of the protease inhibitor compounds described herein are useful in a monotherapy for the prevention and treatment of antiviral, particularly anti-HCV mediated disease. Typically, the pharmaceutical compositions of this invention will be administered from about 1 to about 5 times per day or alternatively, as a continuous infusion. Such administration can be used as a chronic or acute therapy. The amount of active ingredient that may be combined with the carrier materials to produce a single dosage form will vary depending upon the host treated and the particular mode of administration. A typical preparation will contain from about 5% to about 95% active compound (w/w). Preferably, such preparations contain from about 20% to about 80% active compound. As recognized by skilled practitioners, dosages of interferon are typically measured in IU (e.g., about 4 million IU to about 12 million IU).

When the compositions of this invention comprise a combination of VX-950 and one or more additional therapeutic or prophylactic agents, both the compound and the additional agent should be present at dosage levels of between about 10 to 100%, and more preferably between about 10 to 80% of the dosage normally administered in a monotherapy regimen.

The pharmaceutical compositions of this invention may be orally administered in any orally acceptable dosage form including, but not limited to, capsules, tablets, aqueous suspensions or solutions. In the case of tablets for oral use, carriers that are commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added.

Alternatively, the pharmaceutical compositions of this invention may be administered in the form of suppositories for rectal administration. These may be prepared by mixing the agent with a suitable non-irritating excipient which is solid at room temperature but liquid at rectal temperature and therefore will melt in the rectum to release the drug. Such materials include cocoa butter, beeswax and polyethylene glycols.

The pharmaceutical compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract may be effected in a rectal suppository formulation (see above) or in a suitable enema formulation. Topically-transdermal patches may also be used.

For topical applications, the pharmaceutical compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutical compositions may be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with our without a preservative such as benzylalkonium chloride. Alternatively, for ophthalmic uses, the pharmaceutical compositions may be formulated in an ointment such as petrolatum.

The pharmaceutical compositions of this invention may also be administered by nasal aerosol or inhalation. Such compositions are prepared according to techniques well known in the art of pharmaceutical formulation and may be prepared as solutions in saline, employing benzyl alcohol or other suitable preservatives, absorption promoters to

enhance bioavailability, fluorocarbons, and/or other conventional solubilizing or dispersing agents.

Most preferred are pharmaceutical compositions formulated for oral administration.

In another embodiment, the compositions of this invention additionally comprise another anti-viral agent, preferably an anti-HCV agent. Such anti-viral agents include, but are not limited to, immunomodulatory agents, such as α-, β-, and γ-interferons, pegylated derivatized interferon-α compounds, and thymosin; other anti-viral agents, such as ribavirin, amantadine, and telbivudine; other inhibitors of hepatitis C proteases (NS2-NS3 inhibitors and NS3-NS4A inhibitors); inhibitors of other targets in the HCV life cycle, including helicase and polymerase inhibitors; inhibitors of internal ribosome entry; broad-spectrum viral inhibitors, such as IMPDH inhibitors (e.g., compounds of United States Patent 5,807,876, 6,498,178, 6,344,465, 6,054,472, WO 97/40028, WO 98/40381, WO 00/56331, and mycophenolic acid and derivatives thereof, and including, but not limited to VX-497, VX-148, and/or VX-944); or combinations of any of the above. See also W. Markland et al., Antimicrobial & Antiviral Chemotherapy, 44, p. 859 (2000) and U.S. Patent 6,541,496.

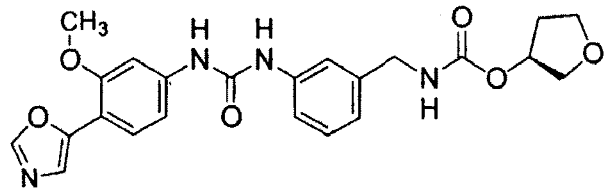

VX-497

The following definitions are used herein (with trademarks referring to products available as of this application's filing date).

"Peg-Intron" means PEG-Intron®, peginteferon alfa-2b, available from Schering Corporation, Kenilworth, NJ;

"Intron" means Intron-A®, interferon alfa-2b available from Schering Corporation, Kenilworth, NJ;

"ribavirin" means ribavirin (1-beta-D-ribofuranosyl-1H-1,2,4-triazole-3-carboxamide, available from ICN Pharmaceuticals, Inc., Costa Mesa, CA; described in the Merck Index, entry 8365, Twelfth Edition; also available as Rebetol® from Schering Corporation, Kenilworth, NJ, or as Copegus® from Hoffmann-La Roche, Nutley, NJ;

"Pagasys" means Pegasys®, peginterferon alfa-2a available Hoffmann-La Roche, Nutley, NJ;

"Roferon" mean Roferon®, recombinant interferon alfa-2a available from Hoffmann-La Roche, Nutley, NJ;

"Berefor" means Berefor®, interferon alfa 2 available from Boehringer Ingelheim Pharmaceutical, Inc., Ridgefield, CT;

Sumiferon®, a purified blend of natural alpha interferons such as Sumiferon available from Sumitomo, Japan;

Wellferon®, interferon alpha nl available from Glaxo_Wellcome LTd., Great Britain;

Alferon®, a mixture of natural alpha interferons made by Interferon Sciences, and available from Purdue Frederick Co., CT;

The term "interferon" as used herein means a member of a family of highly homologous species-specific proteins that inhibit viral replication and cellular proliferation, and modulate immune response, such as interferon alpha, interferon beta, or interferon gamma. The Merck Index, entry 5015, Twelfth Edition.

According to one embodiment of the present invention, the interferon is α-interferon. According to another embodiment, a therapeutic combination of the present invention utilizes natural alpha interferon 2a. Or, the therapeutic combination of the present invention utilizes natural alpha interferon 2b. In another embodiment, the therapeutic combination of the present invention utilizes recombinant alpha interferon 2a or 2b. In yet another embodiment, the interferon is pegylated alpha interferon 2a or 2b. Interferons suitable for the present invention include:

(a) Intron (interferon-alpha 2B, Schering Plough),
(b) Peg-Intron,
(c) Pegasys,
(d) Roferon,
(e) Berofor,
(f) Sumiferon,
(g) Wellferon,
(h) consensus alpha interferon available from Amgen, Inc., Newbury Park, CA,
(i) Alferon;
(j) Viraferon®;
(k) Infergen®.

As is recognized by skilled practitioners, a protease inhibitor would be preferably administered orally. Interferon is not typically administered orally. Nevertheless, nothing herein limits the methods or combinations of this invention to any specific dosage forms or regime. Thus, each component of a combination according to this invention may be administered separately, together, or in any combination thereof.

In one embodiment, the protease inhibitor and interferon are administered in separate dosage forms. In one embodiment, any additional agent is administered as part of a single dosage form with the protease inhibitor or as a separate dosage form. As this invention involves a combination of compounds, the specific amounts of each compound may be dependent on the specific amounts of each other compound in the combination. As recognized by skilled practitioners, dosages of interferon are typically measured in IU (e.g., about 4 million IU to about 12 million IU).

Accordingly, agents (whether acting as an immunomodulatory agent or otherwise) that may be used in combination with a compound of this invention include, but are not limited to, interferon-alph 2B (Intron A, Schering Plough); Rebatron (Schering Plough, Inteferon-alpha 2B + Ribavirin); pegylated interferon alpha (Reddy, K.R. et al. "Efficacy and Safety of Pegylated (40-kd) interferon alpha-2a compared with interferon alpha-2a in noncirrhotic patients with chronic hepatitis C (Hepatology, 33, pp. 433-438 (2001); consensus interferon (Kao, J.H., et al., "Efficacy of Consensus Interferon in the Treatement of Chronic Hepatitis" J. Gastroenterol. Hepatol. 15, pp. 1418-1423 (2000), interferon-alpha 2A (Roferon A; Roche), lymphoblastoid or "natural" interferon; interferon tau (Clayette, P. et al., "IFN-tau, A New Interferon Type I with Antiretroviral activity" Pathol. Biol. (Paris) 47, pp. 553-559 (1999); interleukin 2 (Davis, G.L. et al., "Future Options for the Management of Hepatitis C." Seminars in Liver Disease, 19, pp. 103-112 (1999); Interleukin 6 (Davis et al. "Future Options for the Management of Hepatitis C." Seminars in Liver Disease 19, pp. 103-112 (1999); interleukin 12 (Davis, G.L. et al., "Future Options for the Management of Hepatitis C." Seminars in Liver Disease, 19, pp. 103-112 (1999); Ribavirin; and compounds that enhance the development of type 1 helper T cell response (Davis et al., "Future Options for the Management of Hepatitis C." Seminars in Liver Disease, 19, pp. 103-112 (1999). Interferons may ameliorate viral infections by exerting direct antiviral effects and/or by modifying the immune response to infection. The antiviral effects of interferons are often mediated through inhibition of viral penetration or uncoating, synthesis of viral RNA, translation of viral proteins, and/or viral assembly and release.

Compounds that stimulate the synthesis of interferon in cells (Tazulakhova, E.B. et al., "Russian Experience in Screening, analysis, and Clinical Application of Novel Interferon Inducers" J. Interferon Cytokine Res., 21 pp. 65-73) include, but are not limited to, double stranded RNA, alone or in combination with tobramycin, and Imiquimod (3M Pharmaceuticals; Sauder, D.N. "Immunomodulatory and Pharmacologic Properties of Imiquimod" J. Am. Acad. Dermatol., 43 pp. S6-11 (2000).

Other non-immunomodulatory or immunomodulatory compounds may be used in combination with a compound of this invention including, but not limited to, those specified in WO 02/18369, which is incorporated herein by reference (see, e.g., page 273, lines 9-22 and page 274, line 4 to page 276, line 11, which is incorporated herein by reference in its entirety).

Compounds that stimulate the synthesis of interferon in cells (Tazulakhova et al., J. Interferon Cytokine Res. 21, 65-73)) include, but are not limited to, double stranded RNA, alone or in combination with tobramycin and Imiquimod (3M Pharmaceuticals) (Sauder, J. Am. Arad. Dermatol. 43, S6-11 (2000)).

Other compounds known to have, or that may have, HCV antiviral activity by virtue of non-immunomodulatory mechanisms include, but are not limited to, Ribavirin (ICN Pharmaceuticals); inosine 5'-monophosphate dehydrogenase inhibitors (VX-497 formula provided herein); amantadine and rimantadine (Younossi et al., In Seminars in Liver Disease 19, 95-102 (1999)); LY217896 (U.S. Patent 4,835,168) (Colacino, et al., Antimicrobial Agents & Chemotherapy 34, 2156-2163 (1990)); and 9-Hydroxyimino-6-methoxy-1,4a-dimethyl1,2,3,4,4a,9,10,10a-octahydro-phenanthrene-1-carboxylic acid methyl ester; 6-Methoxy-1,4a dimethyl-9-(4-methyl-piperazin-1-ylimino)-1,2,3,4,4a,9,10,10a-octahydro-phenanthrene-lcarboxylic acid methyl ester-hydrochloride; 1-(2-Chloro-phenyl)-3-(2,2-Biphenyl-ethyl)-urea (U.S. Patent 6,127,422). Formulations, doses, and routes of administration for the foregoing molecules are either taught in the references cited below, or are well-known in the art as disclosed, for example, in F.G. Hayden, in Goodman & Gilman's The Pharmacological Basis of Therapeutics, Ninth Edition, Hardman et al., Eds., McGraw-Hill, New York (1996), Chapter 50, pp. 1191-1223, and the references cited therein. Alternatively, once a compound that exhibits HCV antiviral activity has been identified, a pharmaceutically effective amount of that compound can be determined using techniques that are well-known to the skilled artisan. Note, for example, Benet et al., in Goodman & Gilman's The Phannaeological Basis of Therapeutics, Ninth Edition, Hardman et al., Eds., McGraw-Hill, New York (1996), Chapter 1, pp. 3-27, and the references cited therein. Thus, the appropriate formulations, dose(s) range, and dosing regimens, of such a compound can be easily determined by routine methods. The drug combinations of the present invention can be provided to a cell or cells, or to a human patient, either in separate pharmaceutically acceptable formulations administered simultaneously or sequentially, formulations containing more than one therapeutic agent, or by an assortment of single agent and multiple agent formulations. Regardless of the route of administration, these drug combinations form an anti-HCV effective amount of components.

A large number of other immunomodulators and immununostimulants that:can be used in the methods of the

present invention are currently available and include: AA-2G; adamantylamide dipeptide; adenosine deaminase, Enzon adjuvant, Alliance; adjuvants, Ribi; adjuvants, Vaxcel; Adjuvax; agelasphin-11; AIDS therapy, Chiron; algal glucan, SRI; alganunulin, Anutech; Anginlyc; anticellular factors, Yeda; Anticort; antigastrin-17 immunogen, Ap; antigen delivery system, Vac; antigen formulation, IDBC; antiGnRH immunogen, Aphton; Antiherpin; Arbidol; azarole; Bay-q-8939; Bay-r-1005; BCH-1393; Betafectin; Biostim; BL-001; BL-009; Broncostat; Cantastim; CDRI-84-246; cefodizime; chemokine inhibitors, ICOS; CMV peptides, City of Hope; CN-5888; cytokine-releasing agent, St; DHEAS, Paradigm; DISC TA-HSV; J07B; I01A; I01Z; ditiocarb sodium; ECA-10-142; ELS-1; endotoxin, Novartis; FCE-20696; FCE-24089; FCE-24578; FLT-3 ligand, Immunex; FR-900483; FR-900494; FR-901235; FTS-Zn; G-proteins, Cadus; gludapcin; glutaurine; glycophosphopeptical; GM-2; GM-53; GMDP; growth factor vaccine, EntreM; H-BIG, NABI; H-CIG, NABI; HAB-439; Helicobacter pylori vaccine; herpes-specific immune factor; HIV therapy, United Biomed; HyperGAM+CF; ImmuMax; Immun BCG; immune therapy, Connective; immunomodulator, Evans; immunomodulators, Novacell; imreg-1; imreg-2; Indomune; inosine pranobex; interferon, Dong-A (alpha2); interferon, Genentech (gamma); interferon, Novartis (alpha); interleukin-12, Genetics Ins; interleukin-15, Immunex; interleukin-16, Research Cor; ISCAR-1; J005X; L-644257; licomarasminic acid; LipoTher; LK-409, LK-410; LP-2307; LT (R1926); LW-50020; MAF, Shionogi; MDP derivatives, Merck; met-enkephalin, TNI; methylfurylbutyrolactones; MIMP; mirimostim; mixed bacterial vaccine, Tem, MM-1; moniliastat; MPLA, Ribi; MS-705; murabutide; marabutide, Vacsyn; muramyl dipeptide derivative; muramyl peptide derivatives myelopid; -563; NACOS-6; NH-765; NISV, Proteus; NPT-16416; NT-002; PA-485; PEFA-814; peptides, Scios; peptidoglycan, Pliva; Perthon, Advanced Plant; PGM derivative, Pliva; Pharmaprojects No. 1099; No. 1426; No. 1549; No. 1585; No. 1607; No. 1710; No. 1779; No. 2002; No. 2060; No. 2795; No. 3088; No. 3111; No. 3345; No. 3467; No. 3668; No. 3998; No. 3999; No. 4089; No. 4188; No. 4451; No: 4500; No. 4689; No. 4833; No. 494; No. 5217; No. 530; pidotimod; pimelautide; pinafide; PMD-589; podophyllotoxin, Conpharm; POL-509; poly-ICLC; poly-ICLC, Yamasa Shoyu; PolyA-PolyU; Polysaccharide A; protein A, Berlux Bioscience; PS34WO; Pseudomonas MAbs, Teijin; Psomaglobin; PTL-78419; Pyrexol; pyriferone; Retrogen; Retropep; RG-003; Rhinostat; rifamaxil; RM-06; Rollin; romurtide; RU-40555; RU-41821; Rubella antibodies, ResCo; S-27649; SB-73; SDZ-280-636; SDZ-MRL953; SK&F-107647; SL04; SL05; SM-4333; Solutein; SRI-62-834; SRL-172; ST-570; ST-789; staphage lysate; Stimulon; suppressin; T-150R1; T-LCEF; tabilautide; temurtide; Theradigm-HBV; Theradigm-HBV; Theradigm-HSV; THF, Pharm & Upjohn; THF, Yeda; thymalfasin; thymic hormone fractions; thymocartin; thymolymphotropin; thymopentin; thymopentin analogues; thymopentin, Peptech; thymosin fraction 5, Alpha; thymostimulin; thymotrinan; TMD-232; TO-115; transfer factor, Viragen; tuftsin, Selavo; ubenimex; Ulsastat; ANGG-; CD-4+; Collag+; COLSF+; COM+; DA-A+; GAST-; GF-TH+; GP-120-; IF+; IF-A+; IF-A-2+; IF-B+; IF-G+; IF-G-1B+; IL-2+; IL-12+; IL-15+; IM+; LHRH-; LIPCOR+L LYM-B+; LYM-NK+; LYM-T+; OPI+; PEP+; PHG-MA+; RNA-SYN-; SY-CW-; TH-A-I+; TH-5+; TNF+; UN.

Representative nucleoside and nucleotide compounds useful in the present invention include, but are not limited to: (+) -cis-5-fluoro-1-[2- (hydroxy-methyl) -[1, 3-oxathiolan -5yl]cytosine; (-) -2'-deoxy-3'-thiocytidine-5'-triphospbate (3TC); (-) -cis-5-fluoro-1-[2(hydroxy-methyl) -[I, 3-oxathiolan-5-yl]cytosine (FTC); (-) 2', 3', dideoxy-3'-thiacytidine[(-)-SddC]; 1- (2'-deoxy-2'-fluoro-beta-D-arabinofuranosyl) -5-iodocytosine (FIAC); 1- (2'-deoxy-2'-fluoro-beta-D-arabinofuranosyl) -5-iodocytosine triphosphate (FIACTP); 1- (2'-deoxy-2'-fluoro-beta-D-arabinofuranosyl) -5-methyluracil (FMAU); 1-beta-D-ribofuranosyl-1, 2, 4-triazole-3-carboxamide; 2', 3'-dideoxy-3'-fluoro-5-methyl-dexocytidine (FddMeCyt) ; 2', 3'-dideoxy-3'-chloro-5-methyl-dexocytidine (ClddMeCyt) ; 2', 3'-dideoxy-3'-amino-5-methyl-dexocytidine (AddMeCyt) ; 2', 3'-dideoxy-3'-fluoro-5-methyl-cytidine (FddMeCyt); 2', 3'-dideoxy-3'-chloro-5-methyl-cytidine (ClddMeCyt); 2', 3'-dideoxy-3'-amino-5-methyl-cytidine (AddMeCyt); 2', 3'-dideoxy-3'-fluorothymidine (FddThd); 2', 3'-dideoxy-beta-L-5-fluorocytidine (beta-L-FddC) 2', 3'-dideoxy-beta-L-5-thiacytidine; 2', 3'-dideoxy-beta-L-5-cytidine (beta-L-ddC); 9- (1, 3-dihydroxy-2-propoxymethyl) guanine; 2'-deoxy-3'-thia-5-fluorocytosine; 3'-amino-5-methyl-dexocytidine (AddMeCyt) ;2-amino-1, 9-[(2-hydroxymethyl-1-(hydroxymethyl)ethoxy]methyl]-6H-purin-6-one (gancyclovir) ; 2-[2-(2-amino-9H-purin-9y) ethyl)-1, 3-propandil diacetate (famciclovir) ; 2-amino-1, 9-dihydro-9-[(2-hydroxy-ethoxy) methyl]6H-purin-6-one (acyclovir); 9- (4-hydroxy-3-hydroxymethyl-but-1-yl) guanine (penciclovir); 9- (4-hydroxy-3-hydroxymethyl-but-1-yl) - 6-deoxy-guanine diacetate (famciclovir); 3'-azido-3'-deoxythymidine (AZT) ; 3'-chloro-5-methyl-dexocytidine (ClddMeCyt); 9-(2-phosphonyl-methoxyethyl)-2', 6'-diaminopurine-2', 3'-dideoxyriboside; 9- (2-phosphonylmethoxyethyl) adenine (PMEA) ; acyclovir triphosphate (ACVTP); D-carbocyclic-2'-deoxyguanosine (CdG); dideoxy-cytidine; dideoxy-cytosine (ddC) ; dideoxy-guanine (ddG) ; dideoxy-inosine (ddI) ; E-5- (2-bromovinyl) -2'-deoxyuridine triphosphate; fluoro-arabinofuranosyl-iodouracil; 1- (2'-deoxy-2'-fluoro-1-beta-D-arabinofuranosyl) -5-iodo-uracil (FIAU) ; stavudine; 9-beta-D-arabinofuranosyl-9H-purine-6-amine monohydrate (Ara-A) ; 9-beta-D-arabinofuranosyl-9H-purine-6-amine-5'-monophosphate monohydrate (Ara-AMP); 2-deoxy-3'-thia-5-fluorocytidine; 2', 3'-dideoxy-guanine; and 2', 3'-dideoxy-guanosine.

Synthetic methods for the preparation of nucleosides and nucleotides useful in the present invention are well known in the art as disclosed in Acta Biochim Pol., 43, 25-36 (1996); Swed. Nucleosides Nucleotides 15, 361-378 (1996); Synthesis 12,1465-1479 (1995); Carbohyd. Chem. 27, 242-276 (1995); Chena Nucleosides Nucleotides 3, 421-535 (1994); Ann. Reports in Med. Chena, Academic Press; and Exp. Opin. Invest. Drugs 4, 95-115 (1995). The chemical reactions described in the references cited above are generally disclosed in terms of their broadest application to the

preparation of the compounds of this invention. Occasionally, the reactions may not be applicable as described to each compound included within the scope of compounds disclosed herein. The compounds for which this occurs will be readily recognized by those skilled in the art. In all such cases, either the reactions can be successfully performed by conventional modifications known to those skilled in the art, e.g., by appropriate protection of interfering groups, by changing to alternative conventional reagents, by routine modification of reaction conditions, and the like, or other reactions disclosed herein or otherwise conventional will be applicable to the preparation of the corresponding compounds of this invention. In all preparative methods, all starting materials are known or readily preparable from known starting materials.

While nucleoside analogs are generally employed as antiviral agents as is, nucleotides (nucleoside phosphates) sometimes have to be converted to nucleosides in order to facilitate their transport across cell membranes. An example of a chemically modified nucleotide capable of entering cells is S-1-3-hydroxy-2-phosphonylmethoxypropyl cytosine (HPMPC, Gilead Sciences). Nucleoside and nucleotide compounds used in this invention that are acids can form salts. Examples include salts with alkali metals or alkaline earth metals, such as sodium, potassium, calcium, or magnesium, or with organic bases or basic quaternary ammonium salts.

This invention may also involve administering a cytochrome P450 monooxygenase inhibitor. CYP inhibitors may be useful in increasing liver concentrations and/or increasing blood levels of compounds that are inhibited by CYP.

If an embodiment of this invention involves a CYP inhibitor, any CYP inhibitor that improves the pharmacokinetics of the relevant NS3/4A protease may be used in a method of this invention. These CYP inhibitors include, but are not limited to, ritonavir (WO 94/14436), ketoconazole, troleandomycin, 4-methyl pyrazole, cyclosporin, clomethiazole, cimetidine, itraconazole, fluconazole, miconazole, fluvoxamine, fluoxetine, nefazodone, sertraline, indinavir, nelfinavir, amprenavir, fosamprenavir, saquinavir, lopinavir, delavirdine, erythromycin, VX-944, and VX-497. Preferred CYP inhibitors include ritonavir, ketoconazole, troleandomycin, 4-methyl pyrazole, cyclosporin, and clomethiazole. For preferred dosage forms of ritonavir, see United States Patent 6,037, 157, and the documents cited therein: United States Patent 5,484,801, United States Application 08/402,690, and International Applications WO 95/07696 and WO 95/09614).

Methods for measuring the ability of a compound to inhibit cytochrome P50 monooxygenase activity are known (see US 6,037,157 and Yun, et al. Drug Metabolism & Disposition, vol. 21, pp. 403-407 (1993).

Various published U.S. Patent Applications provide additional teachings of compounds and methods that could be used in combination with VX-950 for the treatment of hepatitis. It is contemplated that any such methods and compositions may be used in combination with the methods and compositions of the present invention. For brevity, the disclosure the disclosures from those publications is referred to be reference to the publication number but it should be noted that the disclosure of the compounds in particular is specifically incorporated herein by reference. Exemplary such publications include U.S. Patent Publication No. 20040058982; U.S. Patent Publication No. 20050192212; U.S. Patent Publication No. 20050080005; U.S. Patent Publication No. 20050062522; U.S. Patent Publication No. 20050020503; U.S. Patent Publication No. 20040229818; U.S. Patent Publication No. 20040229817; U.S. Patent Publication No. 20040224900; U.S. Patent Publication No. 20040186125; U.S. Patent Publication No. 20040171626; U.S. Patent Publication No. 20040110747; U.S. Patent Publication No. 20040072788; U.S. Patent Publication No. 20040067901; U.S. Patent Publication No. 20030191067; U.S. Patent Publication No. 20030187018; U.S. Patent Publication No. 20030186895; U.S. Patent Publication No. 20030181363; U.S. Patent Publication No. 20020147160; U.S. Patent Publication No. 20040082574; U.S. Patent Publication No.. 20050192212; U.S. Patent Publication No. 20050187192; U.S. Patent Publication No. 20050187165; U.S. Patent Publication No. 20050049220.

Immunomodulators, immunostimulants and other agents useful in the combination therapy methods of the present invention can be administered in amounts lower than those conventional in the art. For example, interferon alpha is typically administered to humans for the treatment of HCV infections in an amount of from about 1 x $10^6$ units/person three times per week to about 10 x $10^6$ units/person three times per week (Simon et al., Hepatology 25: 445-448 (1997)). In the methods and compositions of the present invention, this dose can be in the range of from about 0. 1 x $10^6$ units/person three times per week to about 7. 5 x $10^6$ units/person three times per week; more preferably from about 0. 5 x $10^6$ units/person three times per week to about 5 x $10^6$ units/person three times per week; most preferably from about 1 x $10^6$ units/person three times per week to about 3 x $10^6$ units/person three times per week. Due to the enhanced hepatitis C virus antiviral effectiveness of immunomodulators, immunostimulants or other anti-HCV agent in the presence of the HCV serine protease inhibitors of the present invention, reduced amounts of these immunomodulators/immunostimulants can be employed in the treatment methods and compositions contemplated herein. Similarly, due to the enhanced hepatitis C virus antiviral effectiveness of the present HCV serine protease inhibitors in the presence of immunomodulators and immunostimulants, reduced amounts of these HCV serine protease inhibitors can be employed in the methods and compositions contmplated herein. Such reduced amounts can be determined by routine monitoring of hepatitis C virus titers in infected patients undergoing therapy. This can be carried out by, for example, monitoring HCV RNA in patients' serum by slot-blot, dot-blot, or RT-PCR techniques, or by measurement of HCV surface or other antigens. Patients can be similarly monitored during combination therapy employing the HCV serine protease inhibitors disclosed herein and other compounds having anti-HCV activity, for example nucleoside and/or nucleotide antiviral agents, to determine the lowest effective doses of each when used in combination.

In the methods of combination therapy disclosed herein, nucleoside or nucleotide antiviral compounds, or mixtures thereof, can be administered to humans in an amount in the range of from about 0.1 mg/person/day to about 500 mg/person/day; preferably from about 10 mg/person/day to about 300 mg/person/day; more preferably from about 25 mg/person/day to about 200 mg/person/day; even more preferably from about 50 mg/person/day to about 150 mg/person/day; and most preferably in the range of from about 1 mg/person/day to about 50 mg/person/day.

Doses of compounds can be administered to a patient in a single dose or in proportionate doses. In the latter case, dosage unit compositions can contain such amounts of submultiples thereof to make up the daily dose. Multiple doses per day can also increase the total daily dose should this be desired by the person prescribing the drug.

The regimen for treating a patient suffering from a HCV infection with the compounds and/or compositions of the present invention is selected in accordance with a variety of factors, including the age, weight, sex, diet, and medical condition of the patient, the severity of the infection, the route of administration, pharmacological considerations such as the activity, efficacy, pharmacokinetic, and toxicology profiles of the particular compounds employed, and whether a drug delivery system is utilized. Administration of the drug combinations disclosed herein should generally be continued over a period of several weeks to several months or years until virus titers reach acceptable levels, indicating that infection has been controlled or eradicated. Patients undergoing treatment with the drug combinations disclosed herein can be routinely monitored by measuring hepatitis viral RNA in patients' serum by slot-blot, dot-blot, or RT-PCR techniques, or by measurement of hepatitis C viral antigens, such as surface antigens, in serum to determine the effectiveness of therapy. Continuous analysis of the data obtained by these methods permits modification of the treatment regimen during therapy so that optimal amounts of each component in the combination are administered, and so that the duration of treatment can be determined as well. Thus, the treatment regimen/dosing schedule can be rationally modified over the course of therapy so that the lowest amounts of each of the antiviral compounds used in combination which together exhibit satisfactory anti-hepatitis C virus effectiveness are administered, and so that administration of such antiviral compounds in combination is continued only so long as is necessary to successfully treat the infection.

The present invention encompasses the use of the HCV serine protease inhibitors disclosed herein in various combinations with the foregoing and similar types of compounds having anti-HCV activity to treat or prevent HCV infections in patients. For example, one or more HCV serine protease inhibitors can be used in combination with: one or more interferons or interferon derivatives having anti-HCV activity; one or more non-interferon compounds having anti-HCV activity; or one or more interferons or interferon derivatives having anti-HCV activity and one or more non-interferon compounds having anti-HCV activity. When used in combination to treat or prevent HCV infection in a human patient, any of the presently disclosed HCV serine protease inhibitors and foregoing compounds having anti-HCV activity can be present in a pharmaceutically or anti-HCV effective amount. By virtue of their additive or synergistic effects, when used in the combinations described above, each can also be present in a subclinical pharmaceutically effective or anti-HCV effective amount, i.e., an amount that, if used alone, provides reduced pharmaceutical effectiveness in completely inhibiting or reducing the accumulation of HCV virions and/or reducing or ameliorating conditions or symptoms associated with HCV infection or pathogenesis in patients compared to such HCV serine protease inhibitors and compounds having anti-HCV activity when used in pharmaceutically effective amounts. In addition, the present invention encompasses the use of combinations of HCV serine protease inhibitors and compounds having anti-HCV activity as described above to treat or prevent HCV infections, where one or more of these inhibitors or compounds is present in a pharmaceutically effective amount, and the other(s) is(are) present in a subclinical pharmaceutically-effective or anti-HCV effective amount (s) owing to their additive or synergistic effects. As used herein, the term "additive effect" describes the combined effect of two (or more) pharmaceutically active agents that is equal to the sum of the effect of each agent given alone. A synergistic effect is one in which the combined effect of two (or more) pharmaceutically active agents is greater than the sum of the effect of each agent given alone

Upon improvement of a patient's condition, a maintenance dose of a compound, composition or combination of this invention may be administered, if necessary. Subsequently, the dosage or frequency of administration, or both, may be reduced, as a function of the symptoms, to a level at which the improved condition is retained when the symptoms have been alleviated to the desired level, treatment should cease. Patients may, however, require intermittent treatment on a long-term basis upon any recurrence of disease symptoms.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of active ingredients will also depend upon the particular described compound and the presence or absence and the nature of the additional anti-viral agent in the composition.

According to another embodiment, the invention provides a method for treating a patient infected with a virus characterized by a virally encoded serine protease that is necessary for the life cycle of the virus by administering to said patient a pharmaceutically acceptable composition of this invention. Preferably, the methods of this invention are used to treat a patient suffering from a HCV infection. Such treatment may completely eradicate the viral infection or reduce the severity thereof. More preferably, the patient is a human being.

In an alternate embodiment, the methods of this invention additionally comprise the step of administering to said patient an anti-viral agent preferably an anti-HCV agent. Such anti-viral agents include, but are not limited to, immunomodulatory agents, such as α-, β-, and γ-interferons, pegylated derivatized interferon-α compounds, and thymosin; other anti-viral agents, such as ribavirin and amantadine; other inhibitors of hepatitis C proteases (NS2-NS3 inhibitors and NS3-NS4A inhibitors); inhibitors of other targets in the HCV life cycle, including helicase and polymerase inhibitors; inhibitors of internal ribosome entry; broad-spectrum viral inhibitors, such as IMPDH inhibitors (the IMPDH inhibitors disclosed in United States Patent 5,807,876, mycophenolic acid and derivatives thereof); or combinations of any of the above.

Such additional agent may be administered to said patient as part of a single dosage form comprising both a compound of this invention and an additional anti-viral agent. Alternatively the additional agent may be administered separately from the compound of this invention, as part of a multiple dosage form, wherein said additional agent is administered prior to, together with or following a composition comprising a compound of this invention.

In yet another embodiment the present invention provides a method of pre-treating a biological substance intended for administration to a patient comprising the step of contacting said biological substance with a pharmaceutically acceptable composition comprising a compound of this invention. Such biological substances include, but are not limited to, blood and components thereof such as plasma, platelets, subpopulations of blood cells and the like; organs such as kidney, liver, heart, lung, etc; sperm and ova; bone marrow and components thereof, and other fluids to be infused into a patient such as saline, dextrose, etc.

According to another embodiment the invention provides methods of treating materials that may potentially come into contact with a virus characterized by a virally encoded serine protease necessary for its life cycle. This method comprises the step of contacting said material with a compound according to the invention. Such materials include, but are not limited to, surgical instruments and garments (e.g. clothes, gloves, aprons, gowns, masks, eyeglasses, footwear, etc.); laboratory instruments and garments (e.g. clothes, gloves, aprons, gowns, masks, eyeglasses, footwear, etc.); blood collection apparatuses and materials; and invasive devices, such as shunts, stents, etc.

In another embodiment, the compounds of this invention may be used as laboratory tools to aid in the isolation of a virally encoded serine protease. This method comprises the steps of providing a compound of this invention attached to a solid support; contacting said solid support with a sample containing a viral serine protease under conditions that cause said protease to bind to said solid support; and eluting said serine protease from said solid support. Preferably, the viral serine protease isolated by this method is HCV NS3-NS4A protease.

In order that this invention be more fully understood, the following preparative and testing examples are set forth. These examples are for the purpose of illustration only and are not to be construed as limiting the scope of the invention in any way.

## EXAMPLES

The following examples present preferred embodiments and techniques, but are not intended to be limiting. Those of skill in the art will, in light of the present disclosure, appreciate that many changes can be made in the specific materials and methods which are disclosed and still obtain a like or similar result without departing from the spirit and scope of the invention.

### Example 1

### HCV NS3 Protease HPLC Peptide Cleavage Assay

This assay is a modification of that described by Landro et al. [Landro J.A. et al., Biochemistry, 36, pp. 9340-9348 (1997)]. A single peptide substrate (NS5AB), based on the NS5A/NS5B cleavage site for genotype 1a HCV, was used with all proteases. The substrate stock solution (25 mM) was prepared in DMSO containing 0.2M DTT and stored at -20°C. A synthetic peptide cofactor (KK4A) appropriate to each genotype was used as a substitute for the central core region of NS4A. Peptide sequences are shown below. The hydrolysis reaction was performed in a 96-well microtiter plate format using 25 nM to 50 nM HCV NS3 protease in buffer containing 50 mM HEPES pH 7.8, 100 mM NaCl, 20% glycerol, 5 mM DTT and 25 μM KK4A. The final DMSO concentration was no greater than 2% v/v. The reactions were quenched by the addition of 10% trifluoroacetic acid (TFA) to yield a final TFA concentration of 2.5%. Enzymatic activity was assessed by separation of substrate and products on a reverse phase microbore HPLC column (Phenomenex Jupiter 5μ C18 300A column, 150x2.0 mm), which was heated to 40°C using a thermostated column chamber using an Agilent series 1100 instrument with autoinjection and diode array detection at 210 and 280 nm. The flow rate was 0.2 mL/min, with $H_2O$/0.1% TFA (solvent A) and $CH_3CN$/0.1% TFA (solvent B). A linear gradient was used; 5 to 60% solvent B over 12 minutes, then 60% to 100% solvent B over 1 min, 3 min isocratic, followed by 1 min to 5% solvent B and finished with 10 min post time using 5% solvent B isocratic. The SMSY product peak, which typically has a retention

time of 10 min, was analyzed using the data collected at 210 nM.

| Peptide Sequences Used with HCV NS3 protease | | |
|---|---|---|
| Genotype | Peptide | Sequence |
| All | NS5AB | $NH_2$-EDW-(alpha)Abu-CSMSY-COOH |
| 1a | KK4A | $NH_2$-KKGSVVIVGRIVLSGK-COOH |
| 2a | KK4A | $NH_2$-KKGSVSIIGRLHINQRA-COOH |
| 3a | KK4A | $NH_2$-KKGSVVIVGHIELGGKP-COOH |

For determination of the kinetic parameters Km and Vmax, the NS5AB substrate was varied between 3 μM and 200 μM. The ratio of the product peak area to the reaction time yielded a rate of enzyme catalyzed hydrolysis. These rate vs. substrate concentration data points were fit to the Michaelis-Menten equation using non-linear regression. The value of $k_{cat}$ was determined from Vmax using the nominal protease concentration and a fully cleaved substrate peptide as an instrument calibration standard.

| Kinetic Parameters for NS5AB Substrate with HCV NS3 Protease | | |
|---|---|---|
| Genotype | Km (μM) | $k_{cat}$/Km ($M^{-1}sec^{-1}$) |
| 1a | 25 | $3.0 \times 10^4$ |
| 2a | 11 | $3.1 \times 10^4$ |
| 3a | 70 | $5.2 \times 10^3$ |

Example 2

Determination of Potency in HPLC Peptide Cleavage Assay For evaluation of apparent Ki values, all components except the test compound and substrate were pre-incubated for 5 minutes at room temperature. Then, test compound, dissolved in DMSO, was added to the mixture and incubated for 15 minutes at room temperature. The cleavage reaction was initiated by the addition of NS5AB peptide at a concentration equal to Km (11 μM to 70 μM) and incubated at 30°C for fifteen minutes. Seven to eight concentrations of compound were used to titrate enzyme activity for inhibition. Activity vs. inhibitor concentration data points were fit to the Morrison equation describing competitive tight-binding enzyme inhibition using non-linear regression [Sculley, M.J. and Morrison, J.F., Biochim. Biophys. Acta. 874, pp. 44-53 (1986)].

| Apparent Inhibition Constants for VX-950 with HCV NS3 Protease using Peptide Cleavage Assay | |
|---|---|
| Genotype | Ki apparent (nM) |
| 1a | 44 |
| 2a | 40 |
| 3a | 650 |

Example 3

HCV NS3 Protease Fluorescence Peptide Assays

Enzymatic activity was determined using a modification of the assay described by Taliani et al. [Taliani M. et al., Anal. Biochem., 240, pp. 60-67 (1997)]. All reactions were performed in a buffer containing 50 mM HEPES pH 7.8, 100 mM NaCl, 20% glycerol, 5 mM DTT and 25 μM KK4A (Buffer A), using the RET-S1 fluorescent peptide (AnaSpec, San Jose, CA) as substrate. Final DMSO concentrations were maintained at 1-2 % (v/v). Unless otherwise noted, reactions were continuously monitored in a fluorescence microtitre plate reader thermostatted at 30°C, with excitation and emission filters of 355 nm and 495 nm, respectively.

For determination of the kinetic parameters Km and Vmax, the RET-S1 substrate was varied between 6 μM and 200 μM in Buffer A and allowed to react with 5 nM to 10 nM HCV NS3 protease for 5 to 10 minutes. The reactions

were quenched by the addition of 25 μL 10% trifluoroacetic acid (TFA). Enzymatic activity was assessed by separation of substrate and products on a reverse phase microbore HPLC column (Phenomenex Jupiter 5μ C18 300A column, 150×2.0 mm), which was heated to 40 °C using a thermostated column chamber using an Agilent series 1100 instrument with autoinjection and fluorescence detection with excitation at 350 nm and detection at 490 nm. The flow rate was 0.2 mL/min, with $H_2O$/0.1% TFA (solvent A) and $CH_3CN$/0.1% TFA (solvent B). A linear gradient was used; 5 to 100% solvent B over 30 minutes, then 100% to 5% solvent B over 2 min, and finished with 10 min post time using 5% solvent B isocratic. Activity vs. substrate concentration data points were fit to the Michaelis-Menten equation using non-linear regression. The value of $k_{cat}$ was determined from Vmax using the nominal protease concentration and a fully cleaved substrate peptide as an instrument calibration standard.

| Kinetic Parameters for RET-S1 Substrate with HCV NS3 Protease | | |
|---|---|---|
| Genotype | Km (μM) | $k_{cat}$/Km ($M^{-1}sec^{-1}$) |
| 1a | 90 | $1.7 \times 10^5$ |
| 2a | 43 | $6.5 \times 10^4$ |
| 3a | 51 | $2.2 \times 10^4$ |

Example 4

Determination of Potency with Extended Incubation

The inhibition constant for VX-950 and HCV NS3 protease was determined by assaying remaining enzyme activity following an extended preincubation with VX-950. A stock solution of HCV NS3 protease in Buffer A was pre-incubated for 10 minutes at room temperature, then transferred to 30°C. An aliquot of VX-950 dissolved in 100 % DMSO was added to the pre-heated enzyme stock at time zero. The reaction was initiated at time points ranging from 5 to 360 minutes by addition of a 5 μL aliquot of RET-S1 in Buffer A to a 95 μL aliquot of the enzyme-inhibitor mixture, yielding final concentrations of 4 μM RET-S1 and 5 nM to 20 nM HCV NS3 protease. The change in fluorescence was monitored over a 150 second window, and the rate of reaction was determined from a linear regression of the fluorescence vs. time data points. Control rates were determined from a reaction containing neat DMSO. Seven to eight concentrations of compound were used to titrate enzyme activity for inhibition. IC50 values were calculated from activity vs. inhibitor concentration data using a standard logistic 2 parameter fit. Under these assay conditions the IC50 for VX-950 inhibition of HCV NS3 protease following extended incubation is equivalent to the inhibition constant for the tightly bound enzyme/inhibitor complex.

| Inhibition Constants for VX-950 with HCV NS3 Protease following Extended Incubation | |
|---|---|
| Genotype | Ki (nM) |
| 1a | 10 |
| 2a | 37 |
| 3a | 460 |

Example 5

Characterization of Inhibition from Progress Curve

Analysis

The rates of onset of slow binding inhibition were determined by a modification of the method for measurement of progress curves described by Narjes et al. [Narjes F. et al., Biochemistry 39, pp. 1849-1861 (2000)]. A stock solution of HCV NS3 protease in Buffer A was pre-incubated for 10 minutes at room temperature, then transferred to 30°C for an additional 10 minutes. The compound of interest, dissolved in 100% DMSO, was added to a solution of RET-S1 in Buffer A. Compound and substrate were then incubated at 30°C for 10 minutes. The reaction was initiated by addition of an aliquot of pre-heated enzyme stock to the compound-substrate mixture to yield final concentrations of 6 to 12 μM RET-S1 and 0.5 nM to 4 nM HCV NS3 protease. The change in fluorescence was monitored for up to four hours, and

the fluorescence vs. time data points fit to Equation 1 by non-linear regression [Morrison, J.F. and Walsh, C.T., Adv. Enzymol. Relat. Areas Mol. Biol. 61, pp. 201-301 (1988)]. Control rates were determined from a reaction containing neat DMSO.

$$\text{Equation 1: } F(t) = Vs \times t + (Vi - Vs) \times (1-\exp(-k_{obs} \times t))/k_{obs} + C$$

A replot of the $k_{obs}$ values vs. VX-950 concentration allowed the determination of both the second order rate constant for the formation of tightly bound enzyme/inhibitor complex ($k_{on}$) and the first order rate constant for dissociation of the tightly bound enzyme/inhibitor complex ($k_{off}$) by fitting to Equation 2. The inhibition constant for this species was found from the ratio of $k_{off}$ / $k_{on}$ [Morrison, J.F. and Walsh, C.T., Adv. Enzymol. Relat. Areas Mol. Biol. 61, pp. 201-301 (1988)].

$$\text{Equation 2: } k_{obs} = k_{off} + (k_{on} \times [I]) / (1+[S]/Km)$$

| Kinetic Characterization of VX-950 inhibition of HCV NS3 Protease from Progress Curve Analysis | | | |
|---|---|---|---|
| Genotype | $k_{on}$ ($M^{-1}sec^{-1}$) | $k_{off}$ ($sec^{-1}$) | Ki (nM) |
| 1a | $1.3 \times 10^4$ | $1.6 \times 10^{-4}$ | 12 |
| 2a | $2.0 \times 10^4$ | $1.3 \times 10^{-3}$ | 67 |
| 3a | $1.3 \times 10^3$ | $5.7 \times 10^{-4}$ | 440 |

The progress curves obtained above were used to determined the inhibition constant for VX-950 inhibition of HCV NS3 protease through analysis of the remaining enzyme activity at extended reation times. Reaction rates were determined from a linear regression of the fluorescence vs. time data points during the steady-state portion of the reaction. Activity vs. inhibitor concentration data points were fit to the Morrison equation describing competitive tight-binding enzyme inhibition using non-linear regression [Sculley, M.J. and Morrison, J.F., Biochim. Biophys. Acta. 874, pp. 44-53 (1986)].

| Inhibition Constants for VX-950 with HCV NS3 Protease using Steady-State Rates | |
|---|---|
| Genotype | Ki (nM) |
| 1a | 7 |
| 2a | 32 |
| 3a | 270 |

<u>Example 6</u>

<u>Measurement of Dissociation Rates from Enzyme-Inhibitor Complex</u>

A stock solution of HCV NS3 protease in Buffer A was pre-incubated for 10 minutes at room temperature, then transferred to 30 °C for an additional 10 minutes. The compound of interest, dissolved in 100% DMSO, was added to the pre-heated enzyme stock to yield 330 nM to 1600 nM enzyme and 1.0 μM to 6.4 μM inhibitor. This solution was incubated at 30 °C for an extended period to allow the enzyme-inhibitor complex to reach equilibrium. The reaction was initiated by dilution of the enzyme-inhibitor mixture into a solution of RET-S1 in Buffer A at 30 °C. Final concentrations were 0.5 nM to 8 nM HCV NS3 protease, 12 μM RET-S1, and 2 nM to 32 nM inhibitor. The change in fluorescence was monitored for up to four hours, and the fluorescence vs. time data points fit to Equation 2 by non-linear regression. Control rates were determined from a reaction containing neat DMSO. Half-lives of the tightly bound VX-950/HCV NS3 protease complex were determined using Equation 3 [Segel, I.H. Biochemical Calculations, 2nd ed., Wiley & Sons: New

York, p. 228 (1976).

$$\text{Equation 3: } t_{1/2} = 0.693/k_{off}$$

| Dissociation Constants for VX-950/HCV NS3 Protease Complex | | |
|---|---|---|
| Genotype | $k_{off}$ ($sec^{-1}$) | $t_{1/2}$ (min) |
| 1a | $2.0 \times 10^{-4}$ | 58 |
| 2a | $1.3 \times 10^{-3}$ | 9 |
| 3a | $5.6 \times 10^{-4}$ | 21 |

Example 7

HCV Replicon Cell Assay Protocol

Cells were obtained according to the method of Lohmannn et al., Science, 285, pp. 110-113 (1999). Cells containing hepatitis C virus (HCV) replicon were maintained in DMEM containing 10% fetal bovine serum (FBS), 0.25 mg per ml of G418, with appropriate supplements (media A).

On day 1, replicon cell monolayer was treated with a trypsin:EDTA mixture, removed, and then media A was diluted into a final concentration of 100,000 cells per ml wit. 10,000 cells in 100 ul were plated into each well of a 96-well tissue culture plate, and cultured overnight in a tissue culture incubator at 37°C.

On day 2, compounds (in 100% DMSO) were serially diluted into DMEM containing 2% FBS, 0.5% DMSO, with appropriate supplements (media B). The final concentration of DMSO was maintained at 0.5% throughout the dilution series.

Media on the replicon cell monolayer was removed, and then media B containing various concentrations of compounds was added. Media B without any compound was added to other wells as no compound controls.

Cells were incubated with compound or 0.5% DMSO in media B for 48 hours in a tissue culture incubator at 37°C. At the end of the 48-hour incubation, the media was removed, and the replicon cell monolayer was washed once with PBS and stored at -80°C prior to RNA extraction.

Culture plates with treated replicon cell monolayers were thawed, and a fixed amount of another RNA virus, such as Bovine Viral Diarrhea Virus (BVDV) was added to cells in each well. RNA extraction reagents (such as reagents from RNeasy kits) were added to the cells immediately to avoid degradation of RNA. Total RNA was extracted according the instruction of manufacturer with modification to improve extraction efficiency and consistency. Finally, total cellular RNA, including HCV replicon RNA, was eluted and stored at -80°C until further processing.

A Taqman real-time RT-PCR quantification assay was set up with two sets of specific primers and probe. One was for HCV and the other was for BVDV. Total RNA extractants from treated HCV replicon cells was added to the PCR reactions for quantification of both HCV and BVDV RNA in the same PCR well. Experimental failure was flagged and rejected based on the level of BVDV RNA in each well. The level of HCV RNA in each well was calculated according to a standard curve run in the same PCR plate. The percentage of inhibition or decrease of HCV RNA level due to compound treatment was calculated using the DMSO or no compound control as 0% of inhibition. The IC50 (concentration at which 50% inhibition of HCV RNA level is observed) was calculated from the titration curve of any given compound.

VX-950 was found to have an IC50 of 354 nM in this replicon assay.

Example 8

Consensus sequences of the HCV NS3 serine protease domain and NS4A cofactor peptide for genotype 2a, 2b, 3a, or 3b.

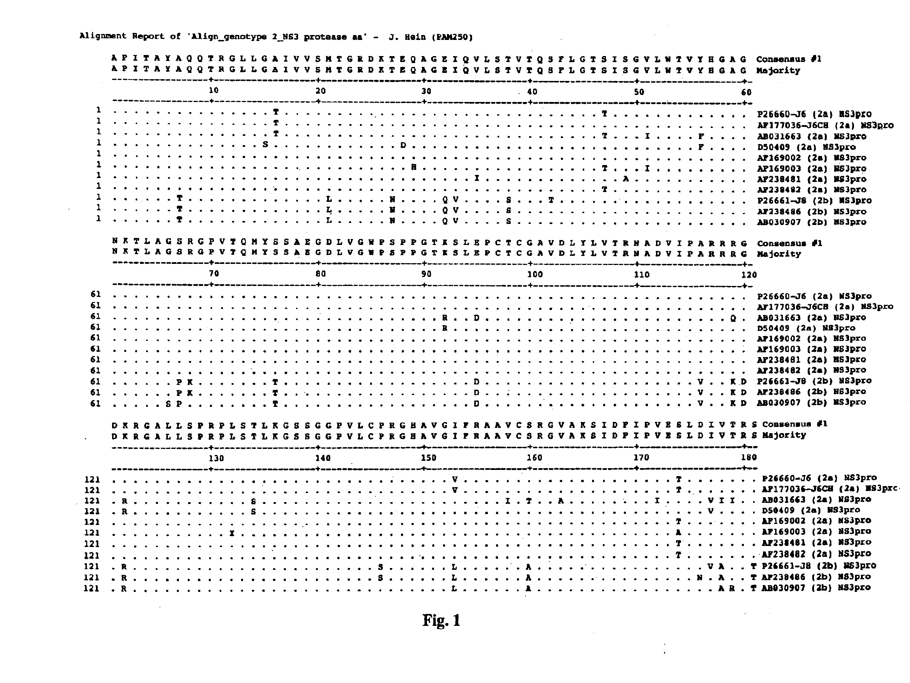

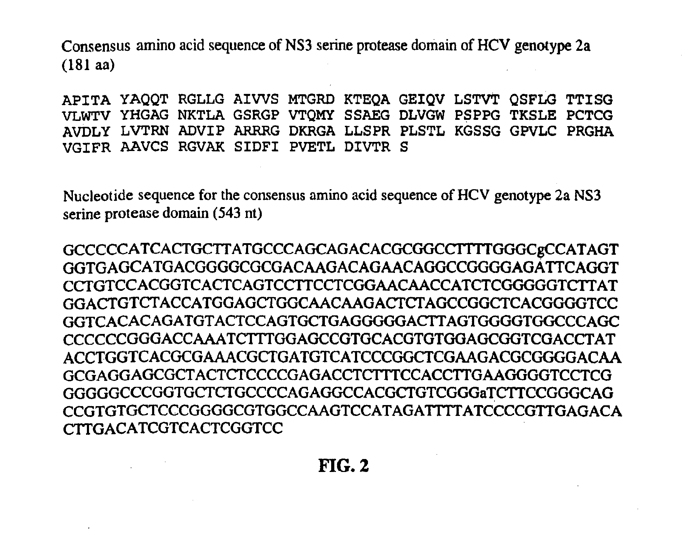

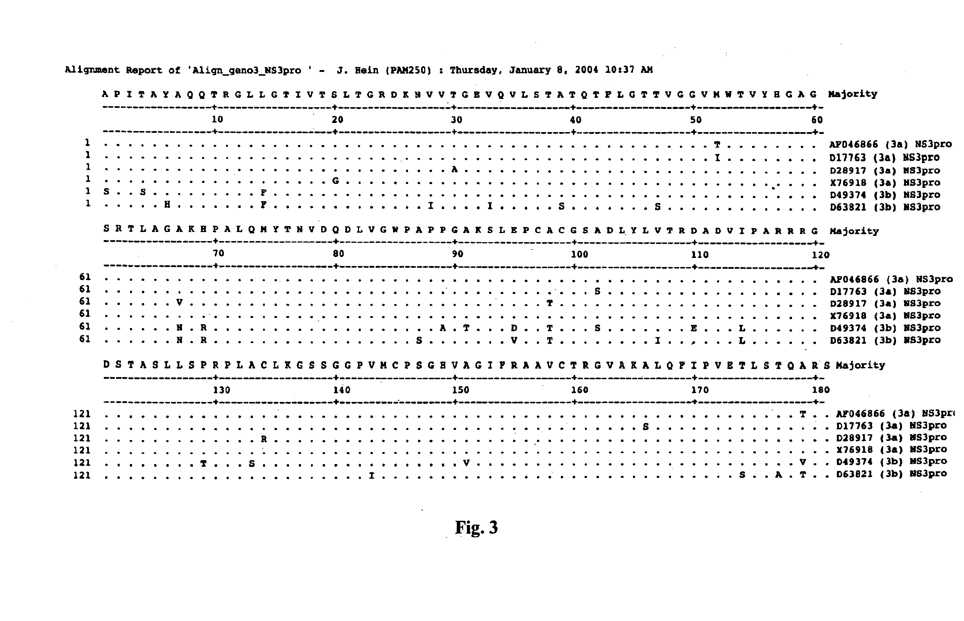

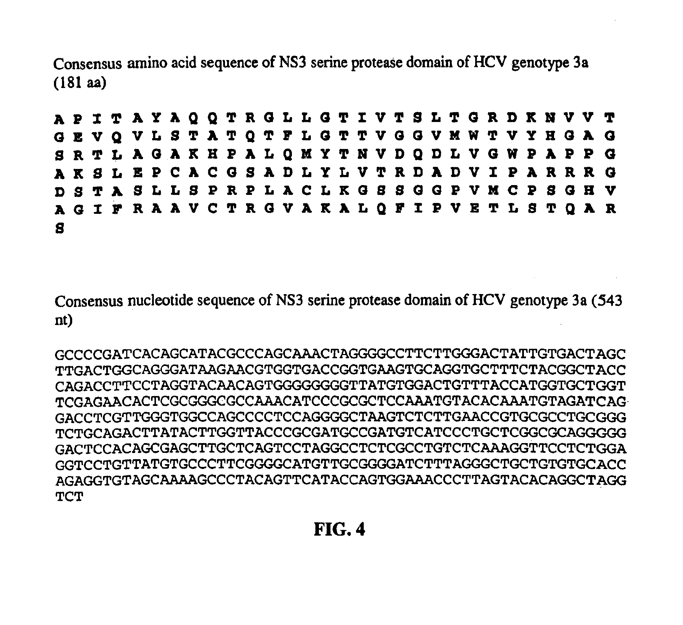

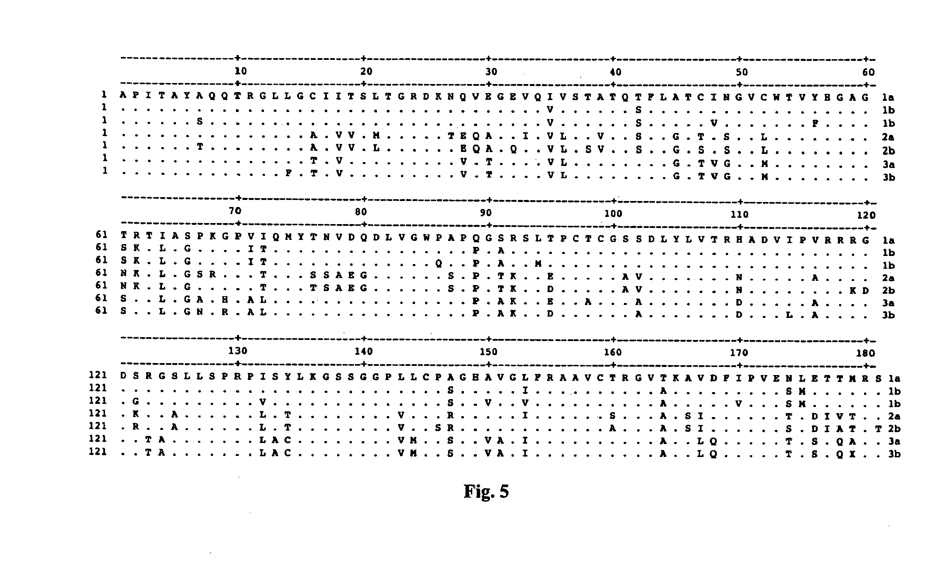

The nucleotide sequences of cDNA fragment covering the NS3 serine protease domain and NS4A cofactor peptide of many HCV isolates were obtained from GenBank and aligned using DNAstar software. These genotype 2 isolates include eight from genotype 2a (GenBank accession code P26660, AF177036, AB031663, D50409, AF169002, AF169003, AF238481, AF238482) and three from genotype 2b (GenBank accession code P26661, AF238486, AB030907). The alignment of amino acid sequence of these eleven genotype 2 HCV NS3 serine protease domains is

shown in Fig. 1. The consensus amino acid and nucleotide sequence of genotype 2a NS3 serine protease domain is shown in Fig. 2. These genotype 3 isolates include four from genotype 3a (GenBank accession code AF046866, D17763, D28917, X76918) and two from genotype 3b (GenBank accession code D49374 and D63821). The alignment of amino acid sequence of these six genotype 3 HCV NS3 serine protease domains is shown in Fig. 3. The consensus amino acid and nucleotide sequence of genotype 3a NS3 serine protease domain is shown in Fig. 4. Finally, an alignment of the consensus amino acid sequence of each genotype or subgenotype 1a, 1b, 2a, 2b, 3a and 3b is shown in Fig. 5.

**Plasmid Construction**. Amino acid and nucleotide sequences of the DNA fragment encoding residues $Ala^{1}$-$Ser^{181}$ of several isolates of genotype 2a or 2b were obtained from GenBank and aligned to identify a consensus sequence for genotype 2a or 2b NS3 serine protease domain. The same applied to genotype 3a or 3b to identify a consensus sequence of genotype 3a or 3b HCV NS3 serine protease domain. The cDNA fragments of these consensus sequences were created by oligonucleotide synthesis (Genscript) using the E. coli optimal codon usage, and then amplified by PCR and subcloned into pBEV11 for expression of the HCV proteins with a C-terminal hexa-histidine tag in E. coli. The amino acid #13 of the HCV NS3 serine protease, Leu was substituted with a Lys for a solubilizing variant. All constructs were confirmed by sequencing.

**Expression and purification of the HCV NS3 serine protease domain.** Each of the expression constructs for the HCV NS3 serine protease domain of genotype 2a or 3a was transformed into BL21/DE3 pLysS E. coli cells (Stratagene). Freshly transformed cells were grown.at 37°C in a BHI medium (Difco Laboratories) supplemented with 100 μg per ml carbenicillin and 35 μg per ml chloramphenicol to an optical density of 0.75 at 600 nM. Induction with 1 mM IPTG was performed for four hours at, 24°C. Cell pastes were harvested by centrifugation and flash frozen at -80°C prior to protein purification. All purification steps were performed at 4°C. For each of the HCV NS3 proteases, 100 g of cell paste was lysed in 1.5 L of buffer A [50 mM HEPES (pH 8.0), 300 mM NaCl, 0.1 % n-octyl-β-D-glucopyranoside, 5 mM β-mercaptoethanol, 10% (v/v) glycerol] and stirred for 30 min. The lysates were homogenized using a Microfluidizer (Microfluidics, Newton, MA), followed by ultra-centrifugation at 54,000x g for 45 min. Imidazole was added to the supernatants to a final concentration of 5 mM along with 2 ml of Ni-NTA resin pre-equilibrated with buffer A containing 5 mM imidazole. The mixtures were rocked for three hours and washed with 20 column volumes of buffer A plus 5 mM imidazole. The HCV NS3 proteins were eluted in buffer A containing 300 mM imidazole. The eluates were concentrated and loaded onto a Hi-Load 16/60 Superdex 200 column, pre-equilibrated with buffer A. The appropriate fractions of the purified HCV proteins were pooled and stored at -80°C.

## EXAMPLE 9

Having determined the consensus domain of the HCV genotypes, the NS3 serine protease domain protein was expressed in E. coli and purified to homogeneity. Enzyme assays for VX-950 were conducted with a KK-4A peptide (Landro et al., 1997 Biochemistry) and a FRET substrate (Taliani et al., 1997 Anal. Biochem.). The Ki* for VX-950 was determined using a steady state method and confirmed by two other methods (extended incubation and progress curves).

Isolation of the consensus domain allowed a determination of the binding characteristics of VX-950 to the domain of HCV-1 as compared to HCV-2. The inventors showed that VX-950 has a several-fold better activity than other inhibitors that been described by those of skill in the art. The data obtained by the inventors show that the binding of VX-950 to the NS3-4A serine protease is a reversible, covalent, competitive, tight and slow binding. As such, this agent has a different mechanism of inhibitory action than other agents that are presently under development. For example, other agents were seen to bind to the protease and the binding was reversible, noncovalent, competitive and tight. More importantly, it was determined that at the binding site of genotype 1 there is a Val-Asp-Gln at residues 78-80 and amino acid 56 whereas in genotype 2 there is a Ala-Glu-Gly. This difference in amino acids at those residues means that there is a lower conformational stability of the loop that is present in the serine protease in the HCV genotype as compared to the stability of the loop in the HCV genotype 1. While the lower conformational stability decreases the binding of some inhibitors, this decrease in conformational stability is expected to have little effect on the binding.of VX-950, making this inhibitor a more potent inhibitor of serine proteases from genotype 2 HCV. Similar results were seen with genotype 3 HCV in which there is a substitution of Gln for the Asp168 that is present genotype 1 HCV.

While a number of embodiments of this invention have been described, it is apparent that the basic examples may be altered to provide other embodiments which utilize the compounds and methods of this invention. Therefore, it will be appreciated that the scope of this invention is to be defined by the appended claims rather than by the specific embodiments that have been represented by way of example above. All cited documents are incorporated herein by reference.

Items

item 1. A method for inhibiting genotype-2 Hepatitis-C virus (HCV) NS3-NS4A protease comprising contacting the protease with VX-950 or a pharmaceutically acceptable salt thereof in an amount effective to inhibit the activity of said protease.

item 2. A method for inhibiting HCV genotype-3 NS3-NS4A protease comprising contacting the protease with VX-950 or a pharmaceutically acceptable salt thereof in an amount effective to inhibit the activity of said protease.

item 3. A method for treating a HCV genotype-2 infection in a patient comprising administering to the patient VX-950 or a pharmaceutically acceptable salt thereof.

item 4. A method for treating a HCV genotype-3 infection in a patient comprising administering to the patient VX-950 or a pharmaceutically acceptable salt thereof.

item 5. The method according to item 3 or item 4, comprising the additional step of administering to said patient an additional agent selected from an immunomodulatory agent; a cytochrome p45 inhibitor, an antiviral agent; a second inhibitor of HCV protease; an inhibitor of another target in the HCV life cycle; or combinations thereof; wherein said additional agent is administered to said patient as part of the same dosage form as VX-950 or as a separate dosage form.

item 6. The method according to item 5, wherein said immunomodulatory agent is α-, β-, or γ-interferon or thymosin; said antiviral agent is ribavarin, amantadine or telbivudine; or said inhibitor of another target in the HCV life cycle is an inhibitor of HCV helicase, polymerase, or metalloprotease.

item 7. The method according to item 5, wherein wherein said cytochrome P-450 inhibitor is ritonavir.

item 8. The method according to item 5, wherein said additional agent is VX-497.

item 9. The method according to item 5, wherein said additional agent is interferon.

item 10. A method of eliminating or reducing genotype-2 or genotype-3 HCV contamination of a biological sample or medical or laboratory equipment, comprising the step of contacting said biological sample or medical or laboratory equipment with VX-950.

item 11. The method according to item 10, wherein said sample or equipment is selected from a body fluid, biological tissue, a surgical instrument, a surgical garment, a laboratory instrument, a laboratory garment, a blood or other body fluid collection apparatus; a blood or other bodily fluid storage material.

item 12. The method according to item 11, wherein said body fluid is blood.

item 13. A composition for inhibiting HCV genotype-2 NS3-NS4A protease comprising i) VX-950, or a pharmaceutically acceptable salt thereof, in an amount effective to inhibit HCV genotype-2 NS3-NS4A protease; and ii) an acceptable carrier, adjuvant or vehicle.

item 14. A composition for inhibiting HCV genotype-3 NS3-NS4A protease comprising i) VX-950, or a pharmaceutically acceptable salt thereof, in an amount effective to inhibit HCV genotype-3 NS3-NS4A protease; and ii) a carrier, adjuvant or vehicle.

item 15. The composition according to item 13 or item 14, wherein said composition is formulated for administration to a patient.

item 16. The composition according to item 15, wherein said carrier, adjuvant or vehicle is a pharmaceutically acceptable carrier, adjuvant or vehicle.

item 17. The composition according to item 16, wherein said composition comprises an additional agent selected from an immunomodulatory agent; a cytochrome p450 inhibitor, an antiviral agent; a second inhibitor of HCV protease; an inhibitor of another target in the HCV life cycle; a cytochrome P-450 inhibitor; or combinations thereof.

item 18. The composition according to item 17, wherein said immunomodulatory agent is α-, β-, or γ-interferon or

thymosin; the antiviral agent is ribavirin, amantadine, or telbivudine; or the inhibitor of another target in the HCV life cycle is an inhibitor of HCV helicase, polymerase, or metalloprotease.

item 19. The composition according to item 17, wherein said cytochrome P-450 inhibitor is ritonavir.

item 20. The composition according to item 17, wherein said additional agent is VX-497.

item 21. The composition according to item 17, wherein said additional agent is interferon.

## Claims

1. A method for inhibiting genotype-2 Hepatitis C virus (HCV) NS3-NS4A protease in vitro comprising contacting the protease with VX-950 or a pharmaceutically acceptable salt thereof in an amount effective to inhibit the activity of said protease.

2. A method for inhibiting HCV genotype-3 NS3-NS4A protease in vitro comprising contacting the protease with VX-950 or a pharmaceutically acceptable salt thereof in an amount effective to inhibit the activity of said protease.

3. VX-950 or a pharmaceutically acceptable salt thereof for use in the treatment of a HCV genotype-2 infection in a patient.

4. VX-950 or a pharmaceutically acceptable salt thereof for use in the treatment of a HCV genotype-3 infection in a patient.

5. VX-950 or a pharmaceutically acceptable salt thereof according to claim 3 or claim 4, wherein the treatment further comprises administration to said patient of an additional agent selected from an immunomodulatory agent, a cytochrome p45 inhibitor, an antiviral agent, a second inhibitor of HCV protease, an inhibitor of another target in the HCV life cycle, or combinations thereof, wherein said additional agent is administered to said patient as part of the same dosage form as VX-950 or as a separate dosage form.

**6.** VX-950 or a pharmaceutically acceptable salt thereof according to claim 5, wherein said immunomodulatory agent is α-, β- or γ-interferon or thymosin, said antiviral agent is ribavarin, amantadine or telbivudine, and said inhibitor of another target in the HCV life cycle is an inhibitor of HCV helicase, polymerase, or metalloprotease.

**7.** VX-950 or a pharmaceutically acceptable salt thereof according to claim 5, wherein said additional agent is VX-497.

**8.** VX-950 or a pharmaceutically acceptable salt thereof according to claim 5, wherein said additional agent is interferon.

Alignment Report of 'Align_genotype 2_NS3 protease aa' - J. Hein (PAM250)

```
    A P I T A Y A Q Q T R G L L G A I V V S M T G R D K T E Q A G E I Q V L S T V T Q S F L G T S I S G V L W T V Y H G A G Consensus #1
    A P I T A Y A Q Q T R G L L G A I V V S M T G R D K T E Q A G E I Q V L S T V T Q S F L G T S I S G V L W T V Y H G A G Majority
                      10                  20                  30                  40                  50                  60
  1 . . . . . . . . . . . . . . . T . . . . . . . . . . . . . . . . . . . . . . . . . . . . . . T . . . . . . . . . . . . . P26660-J6 (2a) NS3pro
  1 . . . . . . . . . . . . . . . T . . . . . . . . . . . . . . . . . . . . . . . . . . . . . . . . . . . . . . . . . . . . . AF177036-J6CH (2a) NS3pro
  1 . . . . . . . . . . . . . . . T . . . . . . . . . . . . . . . . . . . . . . . . . . . . . . T . . . I . . . . F . . . . AB031663 (2a) NS3pro
  1 . . . . . . . . . . . . . . S . . . . . . . . . . . . D . . . . . . . . . . . . . . . . . . . . . . . . . . . F . . . . D50409 (2a) NS3pro
  1 . . . . . . . . . . . . . . . . . . . . . . . . . . . . . . . . . . . . . . . . . . . . . . . . . . . . . . . . . . . . AF169002 (2a) NS3pro
  1 . . . . . . . . . . . . . . . . . . . . . . . . . . . . H . . . . . . . . . . . . . . . . . T . . . I . . . . . . . . . AF169003 (2a) NS3pro
  1 . . . . . . . . . . . . . . . . . . . . . . . . . . . . . . . . . . I . . . . . . . . . . . . . A . . . . . . . . . . . AF238481 (2a) NS3pro
  1 . . . . . . . . . . . . . . . . . . . . . . . . . . . . . . . . . . . . . . . . . . . . . . T . . . . . . . . . . . . . AF238482 (2a) NS3pro
  1 . . . . . . T . . . . . . . . . . . . . L . . . . . N . . . . Q V . . . . S . . . T . . . . . . . . . . . . . . . . . . P26661-J8 (2b) NS3pro
  1 . . . . . . T . . . . . . . . . . . . . L . . . . . N . . . . Q V . . . . S . . . . . . . . . . . . . . . . . . . . . . AF238486 (2b) NS3pro
  1 . . . . . . T . . . . . . . . . . . . . L . . . . . N . . . . Q V . . . . S . . . . . . . . . . . . . . . . . . . . . . AB030907 (2b) NS3pro

    N K T L A G S R G P V T Q M Y S S A E G D L V G W P S P P G T K S L E P C T C G A V D L Y L V T R N A D V I P A R R R G Consensus #1
    N K T L A G S R G P V T Q M Y S S A E G D L V G W P S P P G T K S L E P C T C G A V D L Y L V T R N A D V I P A R R R G Majority
                      70                  80                  90                 100                 110                 120
 61 . . . . . . . . . . . . . . . . . . . . . . . . . . . . . . . . . . . . . . . . . . . . . . . . . . . . . . . . . . . . P26660-J6 (2a) NS3pro
 61 . . . . . . . . . . . . . . . . . . . . . . . . . . . . . . . . . . . . . . . . . . . . . . . . . . . . . . . . . . . . AF177036-J6CH (2a) NS3pro
 61 . . . . . . . . . . . . . . . . . . . . . . . . . . . . . . . R . . D . . . . . . . . . . . . . . . . . . . . . . . . Q . AB031663 (2a) NS3pro
 61 . . . . . . . . . . . . . . . . . . . . . . . . . . . . . . . R . . . . . . . . . . . . . . . . . . . . . . . . . . . . D50409 (2a) NS3pro
 61 . . . . . . . . . . . . . . . . . . . . . . . . . . . . . . . . . . . . . . . . . . . . . . . . . . . . . . . . . . . . AF169002 (2a) NS3pro
 61 . . . . . . . . . . . . . . . . . . . . . . . . . . . . . . . . . . . . . . . . . . . . . . . . . . . . . . . . . . . . AF169003 (2a) NS3pro
 61 . . . . . . . . . . . . . . . . . . . . . . . . . . . . . . . . . . . . . . . . . . . . . . . . . . . . . . . . . . . . AF238481 (2a) NS3pro
 61 . . . . . . . . . . . . . . . . . . . . . . . . . . . . . . . . . . . . . . . . . . . . . . . . . . . . . . . . . . . . AF238482 (2a) NS3pro
 61 . . . . . . P K . . . . . . . T . . . . . . . . . . . . . . . . . . D . . . . . . . . . . . . . . . . . . . . V . . K D P26661-J8 (2b) NS3pro
 61 . . . . . . P K . . . . . . . T . . . . . . . . . . . . . . . . . . D . . . . . . . . . . . . . . . . . . . . V . . K D AF238486 (2b) NS3pro
 61 . . . . . S P . . . . . . . . T . . . . . . . . . . . . . . . . . . D . . . . . . . . . . . . . . . . . . . . V . . K D AB030907 (2b) NS3pro

    D K R G A L L S P R P L S T L K G S S G G P V L C P R G H A V G I F R A A V C S R G V A K S I D P I P V E S L D I V T R S Consensus #1
    D K R G A L L S P R P L S T L K G S S G G P V L C P R G H A V G I F R A A V C S R G V A K S I D P I P V E S L D I V T R S Majority
                     130                 140                 150                 160                 170                 180
121 . . . . . . . . . . . . . . . . . . . . . . . . . . . . . . . . V . . . . . . . . . . . . . . . . . . . . T . . . . . . . P26660-J6 (2a) NS3pro
121 . . . . . . . . . . . . . . . . . . . . . . . . . . . . . . . . V . . . . . . . . . . . . . . . . . . . . T . . . . . . . AF177036-J6CH (2a) NS3pro
121 . R . . . . . . . . . . . S . . . . . . . . . . . . . . . . . . . . . . . I . T . . A . . . . . . . . I . . . . V I I . . AB031663 (2a) NS3pro
121 . R . . . . . . . . . . . S . . . . . . . . . . . . . . . . . . . . . . . . . . . . . . . . . . . . . . . . V . . . . D50409 (2a) NS3pro
121 . . . . . . . . . . . . . . . . . . . . . . . . . . . . . . . . . . . . . . . . . . . . . . . . . . . . . T . . . . . . . AF169002 (2a) NS3pro
121 . . . . . . . . . . . Y . . . . . . . . . . . . . . . . . . . . . . . . . . . . . . . . . . . . . . . . . A . . . . . . . AF169003 (2a) NS3pro
121 . . . . . . . . . . . . . . . . . . . . . . . . . . . . . . . . . . . . . . . . . . . . . . . . . . . . . T . . . . . . . AF238481 (2a) NS3pro
121 . . . . . . . . . . . . . . . . . . . . . . . . . . . . . . . . . . . . . . . . . . . . . . . . . . . . . T . . . . . . . AF238482 (2a) NS3pro
121 . R . . . . . . . . . . . . . . . . . . . . . . . S . . . . . . L . . . . . . A . . . . . . . . . . . . . . . . V A . . T P26661-J8 (2b) NS3pro
121 . R . . . . . . . . . . . . . . . . . . . . . . . S . . . . . . L . . . . . . A . . . . . . . . . . . . . . . N . A . . T AF238486 (2b) NS3pro
121 . R . . . . . . . . . . . . . . . . . . . . . . . . . . . . . . L . . . . . . A . . . . . . . . . . . . . . . . A R . T AB030907 (2b) NS3pro
```

Fig. 1

Consensus amino acid sequence of NS3 serine protease domain of HCV genotype 2a (181 aa)

APITA YAQQT RGLLG AIVVS MTGRD KTEQA GEIQV LSTVT QSFLG TTISG
VLWTV YHGAG NKTLA GSRGP VTQMY SSAEG DLVGW PSPPG TKSLE PCTCG
AVDLY LVTRN ADVIP ARRRG DKRGA LLSPR PLSTL KGSSG GPVLC PRGHA
VGIFR AAVCS RGVAK SIDFI PVETL DIVTR S

Nucleotide sequence for the consensus amino acid sequence of HCV genotype 2a NS3 serine protease domain (543 nt)

GCCCCCATCACTGCTTATGCCCAGCAGACACGCGGCCTTTTGGGCgCCATAGT
GGTGAGCATGACGGGGCGCGACAAGACAGAACAGGCCGGGGAGATTCAGGT
CCTGTCCACGGTCACTCAGTCCTTCCTCGGAACAACCATCTCGGGGGTCTTAT
GGACTGTCTACCATGGAGCTGGCAACAAGACTCTAGCCGGCTCACGGGGTCC
GGTCACACAGATGTACTCCAGTGCTGAGGGGGACTTAGTGGGGTGGCCCAGC
CCCCCCGGGACCAAATCTTTGGAGCCGTGCACGTGTGGAGCGGTCGACCTAT
ACCTGGTCACGCGAAACGCTGATGTCATCCCGGCTCGAAGACGCGGGGACAA
GCGAGGAGCGCTACTCTCCCCGAGACCTCTTTCCACCTTGAAGGGGTCCTCG
GGGGGCCCGGTGCTCTGCCCCAGAGGCCACGCTGTCGGGaTCTTCCGGGCAG
CCGTGTGCTCCCGGGGCGTGGCCAAGTCCATAGATTTTATCCCCGTTGAGACA
CTTGACATCGTCACTCGGTCC

**FIG. 2**

Alignment Report of 'Align_geno3_NS3pro ' - J. Hein (PAM250) : Thursday, January 8, 2004 10:37 AM

```
      A P I T A Y A Q Q T R G L L G T I V T S L T G R D K N V V T G E V Q V L S T A T Q T F L G T T V G G V M W T V Y H G A G  Majority
      ------------------+-------------------+-------------------+-------------------+-------------------+-------------------+-
                       10                  20                  30                  40                  50                  60
      ------------------+-------------------+-------------------+-------------------+-------------------+-------------------+-
  1   . . . . . . . . . . . . . . . . . . . . . . . . . . . . . . . . . . . . . . . . . . . . . . . . . . . T . . . . . . . .  AF046866 (3a) NS3pro
  1   . . . . . . . . . . . . . . . . . . . . . . . . . . . . . . . . . . . . . . . . . . . . . . . . . . . I . . . . . . . .  D17763 (3a) NS3pro
  1   . . . . . . . . . . . . . . . . . . . . . . . . . . . . . A . . . . . . . . . . . . . . . . . . . . . . . . . . . . . .  D28917 (3a) NS3pro
  1   . . . . . . . . . . . . . . . . . . . G . . . . . . . . . . . . . . . . . . . . . . . . . . . . . . . . . . . . . . . .  X76918 (3a) NS3pro
  1   S . . S . . . . . . . . . F . . . . . . . . . . . . . . . . . . . . . . . . . . . . . . . . . . . . . . . . . . . . . .  D49374 (3b) NS3pro
  1   . . . . . H . . . . . . . F . . . . . . . . . . . . . I . . . . I . . . . . S . . . . . . . S . . . . . . . . . . . . .  D63821 (3b) NS3pro

      S R T L A G A K H P A L Q M Y T N V D Q D L V G W P A P P G A K S L E P C A C G S A D L Y L V T R D A D V I P A R R R G  Majority
      ------------------+-------------------+-------------------+-------------------+-------------------+-------------------+-
                       70                  80                  90                 100                 110                 120
      ------------------+-------------------+-------------------+-------------------+-------------------+-------------------+-
 61   . . . . . . . . . . . . . . . . . . . . . . . . . . . . . . . . . . . . . . . . . . . . . . . . . . . . . . . . . . . .  AF046866 (3a) NS3pro
 61   . . . . . . . . . . . . . . . . . . . . . . . . . . . . . . . . . . . . . . . . . S . . . . . . . . . . . . . . . . . .  D17763 (3a) NS3pro
 61   . . . . . . V . . . . . . . . . . . . . . . . . . . . . . . . . . . . . . T . . . . . . . . . . . . . . . . . . . . . .  D28917 (3a) NS3pro
 61   . . . . . . . . . . . . . . . . . . . . . . . . . . . . . . . . . . . . . . . . . . . . . . . . . . . . . . . . . . . .  X76918 (3a) NS3pro
 61   . . . . . . N . R . . . . . . . . . . . . . . . . . . . A . T . . . D . . T . . . S . . . . . . . E . . . L . . . . . .  D49374 (3b) NS3pro
 61   . . . . . . N . R . . . . . . . . . . . . . . . . . S . . . . . . . V . . T . . . . . . . . I . . . . . . L . . . . . .  D63821 (3b) NS3pro

      D S T A S L L S P R P L A C L K G S S G G P V M C P S G H V A G I P R A A V C T R G V A K A L Q F I P V E T L S T Q A R S  Majority
      ------------------+-------------------+-------------------+-------------------+-------------------+-------------------+-
                      130                 140                 150                 160                 170                 180
      ------------------+-------------------+-------------------+-------------------+-------------------+-------------------+-
121   . . . . . . . . . . . . . . . . . . . . . . . . . . . . . . . . . . . . . . . . . . . . . . . . . . . . . . . . T . .  AF046866 (3a) NS3pro
121   . . . . . . . . . . . . . . . . . . . . . . . . . . . . . . . . . . . . . . . . . . . . . S . . . . . . . . . . . . . . .  D17763 (3a) NS3pro
121   . . . . . . . . . . . . . R . . . . . . . . . . . . . . . . . . . . . . . . . . . . . . . . . . . . . . . . . . . . . . .  D28917 (3a) NS3pro
121   . . . . . . . . . . . . . . . . . . . . . . . . . . . . . . . . . . . . . . . . . . . . . . . . . . . . . . . . . . . . .  X76918 (3a) NS3pro
121   . . . . . . . . T . . . S . . . . . . . . . . . . . . . . . V . . . . . . . . . . . . . . . . . . . . . . . . . . . V . .  D49374 (3b) NS3pro
121   . . . . . . . . . . . . . . . . . . . . . . I . . . . . . . . . . . . . . . . . . . . . . . . . . . . . S . . A . T . .  D63821 (3b) NS3pro
```

**Fig. 3**

Consensus amino acid sequence of NS3 serine protease domain of HCV genotype 3a (181 aa)

A P I T A Y A Q Q T R G L L G T I V T S L T G R D K N V V T
G E V Q V L S T A T Q T F L G T T V G G V M W T V Y H G A G
S R T L A G A K H P A L Q M Y T N V D Q D L V G W P A P P G
A K S L E P C A C G S A D L Y L V T R D A D V I P A R R R G
D S T A S L L S P R P L A C L K G S S G G P V M C P S G H V
A G I F R A A V C T R G V A K A L Q F I P V E T L S T Q A R
S

Consensus nucleotide sequence of NS3 serine protease domain of HCV genotype 3a (543 nt)

GCCCCGATCACAGCATACGCCCAGCAAACTAGGGGCCTTCTTGGGACTATTGTGACTAGC
TTGACTGGCAGGGATAAGAACGTGGTGACCGGTGAAGTGCAGGTGCTTTCTACGGCTACC
CAGACCTTCCTAGGTACAACAGTGGGGGGGTTATGTGGACTGTTTACCATGGTGCTGGT
TCGAGAACACTCGCGGGCGCCAAACATCCCGCGCTCCAAATGTACACAAATGTAGATCAG
GACCTCGTTGGGTGGCCAGCCCCTCCAGGGGCTAAGTCTCTTGAACCGTGCGCCTGCGGG
TCTGCAGACTTATACTTGGTTACCCGCGATGCCGATGTCATCCCTGCTCGGCGCAGGGGG
GACTCCACAGCGAGCTTGCTCAGTCCTAGGCCTCTCGCCTGTCTCAAAGGTTCCTCTGGA
GGTCCTGTTATGTGCCCTTCGGGGCATGTTGCGGGGATCTTTAGGGCTGCTGTGTGCACC
AGAGGTGTAGCAAAAGCCCTACAGTTCATACCAGTGGAAACCCTTAGTACACAGGCTAGG
TCT

**FIG. 4**

```
                  10        20        30        40        50        60
  1 APITAYAQQTRGLLGCIITSLTGRDKNQVEGEVQIVSTATQTFLATCINGVCWTVYHGAG 1a
  1 ..................................V......S.................. 1b
  1 ......S...........................V......S.....V........P... 1b
  1 ...............A.VV.M.....TEQA..I.VL..V..S..G.T.S..L........ 2a
  1 ......T........A.VV.L......EQA.Q..VL.SV..S..G.S.S..L........ 2b
  1 ...............T.V.........V.T....VL........G.TVG..M........ 3a
  1 .............F.T.V.........V.T....VL........G.TVG..M........ 3b

                  70        80        90       100       110       120
 61 TRTIASPKGPVIQMYTNVDQDLVGWPAPQGSRSLTPCTCGSSDLYLVTRHADVIPVRRRG 1a
 61 SK.L.G....IT................P.A............................. 1b
 61 SK.L.G....IT.............Q..P.A..M.......................... 1b
 61 NK.L.GSR...T...SSAEG......S.P.TK..E.....AV.......N.....A.... 2a
 61 NK.L.G.....T...TSAEG......S.P.TK..D.....AV.......N........KD 2b
 61 S..L.GA.H.AL................P.AK..E..A...A.......D.....A.... 3a
 61 S..L.GN.R.AL................P.AK..D......A.......D...L.A.... 3b

                 130       140       150       160       170       180
121 DSRGSLLSPRPISYLKGSSGGPLLCPAGHAVGLFRAAVCTRGVTKAVDFIPVENLETTMRS 1a
121 ..........................S.....I..........A.........SM...... 1b
121 .G.........V..............S..V..V..........A.....V...SM...... 1b
121 .K..A......L.T........V...R.....I......S...A.SI......T.DIVT.. 2a
121 .R..A......L.T........V..SR.............A..A.SI......S.DIAT.T 2b
121 ..TA.......LAC........VM..S..VA.I..........A..LQ.....T.S.QA.. 3a
121 ..TA.......LAC........VM..S..VA.I..........A..LQ.....T.S.QX.. 3b
```

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0218369 A
- WO 0208244 A
- WO 0009558 A
- WO 0009543 A
- WO 9964442 A
- WO 9907733 A
- WO 9907734 A
- WO 9950230 A
- WO 9846630 A
- WO 9817679 A
- WO 9743310 A
- US 5990276 A
- WO 02018369 A
- US 5807876 A
- US 6498178 B
- US 6344465 B
- US 6054472 A
- WO 9740028 A
- WO 9840381 A
- WO 0056331 A
- US 6541496 B
- US 4835168 A
- US 6127422 A
- WO 9414436 A
- US 6037157 A
- US 5484801 A
- US 08402690 B
- WO 9507696 A
- WO 9509614 A
- US 20040058982 A
- US 20050192212 A
- US 20050080005 A
- US 20050062522 A
- US 20050020503 A
- US 20040229818 A
- US 20040229817 A
- US 20040224900 A
- US 20040186125 A
- US 20040171626 A
- US 20040110747 A
- US 20040072788 A
- US 20040067901 A
- US 20030191067 A
- US 20030187018 A
- US 20030186895 A
- US 20030181363 A
- US 20020147160 A
- US 20040082574 A
- US 20050187192 A
- US 20050187165 A
- US 20050049220 A


### Non-patent literature cited in the description


- **A. ALBERTI et al.** Natural History of Hepatitis C. *J. Hepatology,* 1999, vol. 31, 17-24
- **M.J. ALTER et al.** The Epidemiology of Viral Hepatitis in the United States. *Gastroenterol. Clin. North Am.,* 1994, vol. 23, 437-455
- **M. J. ALTER.** Hepatitis C Virus Infection in the United States. *J. Hepatology,* 1999, vol. 31, 88-91
- **S. IWARSON.** The Natural Course of Chronic Hepatitis. *FEMS Microbiology Reviews,* 1994, vol. 14, 201-204
- **D. LAVANCHY.** Global Surveillance and Control of Hepatitis C. *J. Viral Hepatitis,* 1999, vol. 6, 35-47
- **M.C. KEW.** Hepatitis C and Hepatocellular Carcinoma. *FEMS Microbiology Reviews,* 1994, vol. 14, 211-220
- **I. SAITO et al.** Hepatitis C Virus Infection is Associated with the Development of Hepatocellular Carcinoma. *Proc. Natl. Acad. Sci. USA,* 1990, vol. 87, 6547-6549
- **Q.L. CHOO et al.** Genetic Organization and Diversity of the Hepatitis C Virus. *Proc. Natl. Acad. Sci. USA,* 1991, vol. 88, 2451-2455
- **N. KATO et al.** Molecular Cloning of the Human Hepatitis C Virus Genome From Japanese Patients with Non-A, Non-B Hepatitis. *Proc. Natl. Acad. Sci. USA,* 1990, vol. 87, 9524-9528
- **TAKAMIZAWA et al.** Structure and Organization of the Hepatitis C Virus Genome Isolated From Human Carriers. *J. Virol.,* 1991, vol. 65, 1105-1113
- **R. BARTENSCHLAGER et al.** Nonstructural Protein 3 of the Hepatitis C Virus Encodes a Serine-Type Proteinase Required for Cleavage at the NS3/4 and NS4/5 Junctions. *J. Virol.,* 1993, vol. 67, 3835-3844

- **A. GRAKOUI et al.** Characterization of the Hepatitis C Virus-Encoded Serine Proteinase: Determination of Proteinase-Dependent Polyprotein Cleavage Sites. *J. Virol.,* 1993, vol. 67, 2832-2843
- **A. GRAKOUI et al.** Expression and Identification of Hepatitis C Virus Polyprotein Cleavage Products. *J. Virol.,* 1993, vol. 67, 1385-1395
- **L. TOMEI et al.** NS3 is a serine protease required for processing of hepatitis C virus polyprotein. *J. Virol.,* 1993, vol. 67, 4017-4026
- **A.A. KOLYKHALOV et al.** Hepatitis C virus-encoded enzymatic activities and conserved RNA elements in the 3' nontranslated region are essential for virus replication in vivo. *J. Virol.,* vol. 74, 2046-2051
- **C. LIN et al.** Hepatitis C Virus NS3 Serine Proteinase: Trans-Cleavage Requirements and Processing Kinetics. *J. Virol.,* 1994, vol. 68, 8147-8157
- **M. LLINAS-BRUNET et al.** *Bioorg. Med. Chem. Lett.,* 1998, vol. 8, 1713-18
- **W. HAN et al.** *Bioorg. Med. Chem. Lett.,* 2000, vol. 10, 711-13
- **R. DUNSDON et al.** *Bioorg. Med. Chem. Lett.,* 2000, vol. 10, 1571-79
- **M. LLINAS-BRUNET et al.** *Bioorg. Med. Chem. Lett.,* 2000, vol. 10, 2267-70
- **S. LAPLANTE et al.** *Bioorg. Med. Chem. Lett.,* 2000, vol. 10, 2271-74
- **M. A. WLAKER et al.** Hepatitis C Virus: An Overview of Current Approaches and Progress. *DDT,* 1999, vol. 4, 518-29
- **D. MORADPOUR et al.** Current and Evolving Therapies for Hepatitis C. *Eur. J. Gastroenterol. Hepatol.,* 1999, vol. 11, 1199-1202
- **H. L. A. JANSSEN et al.** Suicide Associated with Alfa-Interferon Therapy for Chronic Viral Hepatitis. *J. Hepatol.,* 1994, vol. 21, 241-243
- **P.F. RENAULT et al.** Side Effects of Alpha Interferon. *Seminars in Liver Disease,* 1989, vol. 9, 273-277
- **O. WEILAND.** Interferon Therapy in Chronic Hepatitis C Virus Infection. *FEMS Microbiol. Rev.,* 1994, vol. 14, 279-288
- **J.G. MCHUTCHISON et al.** *N. Engl. J. Med.,* 1998, vol. 339, 1485-1492
- **G.L. DAVIS et al.** *N. Engl. J. Med.,* 1998, vol. 339, 1493-1499
- **W. MARKLAND et al.** *Antimicrobial & Antiviral Chemotherapy,* 2000, vol. 44, 859
- **REDDY, K.R. et al.** Efficacy and Safety of Pegylated (40-kd) interferon alpha-2a compared with interferon alpha-2a in noncirrhotic patients with chronic hepatitis C. *Hepatology,* 2001, vol. 33, 433-438
- **KAO, J.H. et al.** Efficacy of Consensus Interferon in the Treatement of Chronic Hepatitis. *J. Gastroenterol. Hepatol.,* 2000, vol. 15, 1418-1423
- **CLAYETTE, P. et al.** IFN-tau, A New Interferon Type I with Antiretroviral activity. *Pathol. Biol.,* 1999, vol. 47, 553-559
- **DAVIS, G.L. et al.** Future Options for the Management of Hepatitis C. *Seminars in Liver Disease,* 1999, vol. 19, 103-112
- **DAVIS et al.** Future Options for the Management of Hepatitis C. *Seminars in Liver Disease,* 1999, vol. 19, 103-112
- **TAZULAKHOVA, E.B. et al.** Russian Experience in Screening, analysis, and Clinical Application of Novel Interferon Inducers. *J. Interferon Cytokine Res.,* vol. 21, 65-73
- **SAUDER, D.N.** Immunomodulatory and Pharmacologic Properties of Imiquimod. *J. Am. Acad. Dermatol.,* 2000, vol. 43, S6-11
- **TAZULAKHOVA et al.** *J. Interferon Cytokine Res.,* vol. 21, 65-73
- **SAUDER.** *J. Am. Arad. Dermatol.,* 2000, vol. 43, 6-11
- **YOUNOSSI et al.** *In Seminars in Liver Disease,* 1999, vol. 19, 95-102
- **COLACINO et al.** *Antimicrobial Agents & Chemotherapy,* 1990, vol. 34, 2156-2163
- **F.G. HAYDEN et al.** Goodman & Gilman's The Pharmacological Basis of Therapeutics. McGraw-Hill, 1996, 1191-1223
- **BENET et al.** Goodman & Gilman's The Phannaeological Basis of Therapeutics. McGraw-Hill, 1996, 3-27
- *Acta Biochim Pol.,* 1996, vol. 43, 25-36
- *Swed. Nucleosides Nucleotides,* 1996, vol. 15, 361-378
- *Synthesis,* 1995, vol. 12, 1465-1479
- *Carbohyd. Chem.,* 1995, vol. 27, 242-276
- *Chena Nucleosides Nucleotides,* 1994, vol. 3, 421-535
- Ann. Reports in Med. Chena. Academic Press
- *Exp. Opin. Invest. Drugs,* 1995, vol. 4, 95-115
- **YUN et al.** *Drug Metabolism & Disposition,* 1993, vol. 21, 403-407
- **SIMON et al.** *Hepatology,* 1997, vol. 25, 445-448
- **LANDRO J.A. et al.** *Biochemistry,* 1997, vol. 36, 9340-9348
- **SCULLEY, M.J. ; MORRISON, J.F.** *Biochim. Biophys. Acta,* 1986, vol. 874, 44-53
- **TALIANI M. et al.** *Anal. Biochem.,* 1997, vol. 240, 60-67
- **NARJES F. et al.** *Biochemistry,* 2000, vol. 39, 1849-1861
- **MORRISON, J.F. ; WALSH, C.T.** *Adv. Enzymol. Relat. Areas Mol. Biol.,* 1988, vol. 61, 201-301
- **SCULLEY, M.J. ; MORRISON, J.F.** *Biochim. Biophys. Acta.,* 1986, vol. 874, 44-53
- **SEGEL, I.H.** Biochemical Calculations. Wiley & Sons, 1976, 228
- **LOHMANNN et al.** *Science,* 1999, vol. 285, 110-113
- **LANDRO et al.** *Biochemistry,* 1997

- **TALIANI et al.** *Anal. Biochem.*, 1997